# EUROPÄISCHE PATENTANMELDUNG

(11) **EP 4 700 514 A1**
(43) Veröffentlichungstag der Anmeldung: **25.02.2026**
(21) Anmeldenummer: 25195799.9
(22) Anmeldetag: 13.08.2025
(51) Int. Cl.: G05B 19/418, G01N 21/90

(54) **VERFAHREN ZUM BETREIBEN EINER BEHÄLTNISBEHANDLUNGSANLAGE UND STEUERVORRICHTUNG FÜR EINE BEHÄLTNISBEHANDLUNGSANLAGE**

(30) Priorität: 22.08.2024 DE 102024124078
(71) Anmelder: Krones AG, 93073 Neutraubling (DE)
(72) Erfinder: Hewicker, Alexander, 93073 Neutraubling (DE)
(74) Vertreter: Bittner, Bernhard

(57) **Zusammenfassung**

Verfahren zum Betreiben einer Behältnisbehandlungsanlage zur Behandlung einer Vielzahl von Behältnisteilen für Behältnisse, Kunststoffbehältnisse und/oder Flaschen, wobei eine Transporteinrichtung die Behältnisteilen als Behältnisteil-Strom entlang eines Transportpfads von einer Behandlungseinrichtung der Behältnisbehandlungsanlage zu einer weiteren Behandlungseinrichtung der Behältnisbehandlungsanlage transportiert, wobei zur Durchführung einer Behältnisinspektionsaufgabe eine Sensoreinrichtung, insbesondere ortsaufgelöste, Sensordaten und Kamerabilder in Bezug auf die Behältnisteile, optisch, erfasst und eine Echtzeit-Auswertungseinrichtung die ortsaufgelösten Sensordaten in Echtzeit mittels eines Behältnisinspektions-Modell maschinellen Lernens, welches einen Satz Parameter umfasst, welche auf Werte eingestellt sind, die als Ergebnis eines maschinellen Lernverfahrens gelernt wurden, auswertet, dadurch gekennzeichnet, dass ein auf einem maschinellen Lernverfahren basierender Satz von Behältnisteil-Merkmalen vorgegeben ist und die erfassten, ortsaufgelösten, Sensordaten in Bezug auf eine, von der Behältnisinspektionsaufgabe verschiedene Anlagen-Inspektionsaufgabe basierend auf dem vorgegebenen Satz von Behältnisteil-Merkmalen ausgewertet werden, wobei in Abhängigkeit des Inspektionsergebnisses der durchgeführten Anlagen-Inspektionsaufgabe eine Anlageninspektionsgröße ermittelt wird, oder eine Ähnlichkeitsgröße ermittelt wird, welche charakteristisch ist für die Ähnlichkeit der ortsaufgelösten Sensordaten zu Referenzdaten, und wobei diese Größen zur Steuerung und/oder Regelung der Behältnisbehandlungsanlage bereitgestellt werden.

## Beschreibung

Die vorliegende Erfindung bezieht sich auf ein Verfahren zum Betreiben einer Behältnisbehandlungsanlage zur Behandlung einer Vielzahl von Behältnisteilen für Behältnisse und bevorzugt für Kunststoffbehältnisse und/oder Flaschen, sowie eine Steuervorrichtung für eine Behältnisbehandlungsanlage und eine Behältnisbehandlungsanlage. Dabei ist eine Transporteinrichtung vorgesehen, welche die Vielzahl von Behältnisteilen als Behältnisteil-Strom entlang eines vorgegebenen Transportpfads transportiert.

Bei den Behältnissen handelt es sich bevorzugt um Kunststoffbehältnisse (insbesondere PET-Behältnisse), Behältnisse deren Hauptbestandteil aus Pulpe besteht und/oder Glasbehältnisse und/oder Dosen. Bei den Behältnissen kann es sich dabei um Behältnisse aus der Getränke- und/oder Lebensmittel- und/oder Kosmetikindustrie und/oder Pharmaindustrie handeln.

Aus der DE 10 2021 133 164 B3 ist ein Verfahren zum Durchführen eines Einstellbetriebs einer Behältnisinspektionsvorrichtung bekannt. Darin erfasst eine Sensoreinrichtung ortsaufgelöste Sensordaten in Bezug auf die zu inspizierenden Behältnisse und eine Echtzeit-Auswertungseinrichtung wertet die ortsaufgelösten Sensordaten der einzelnen inspizierten Behältnisse mithilfe eines einstellbaren Echtzeit-Behältnisinspektions-Modells in Echtzeit aus. Weiter wird eine Vielzahl von ortsaufgelösten Sensordaten auf einer nicht-flüchtigen Speichereinrichtung bereitgestellt. In einem Einstellbetrieb ruft eine Einstelleinrichtung die abgelegte Vielzahl von ortsaufgelösten Sensordaten ab und bewertet auf Grundlage der abgerufenen Vielzahl von ortsaufgelösten Sensordaten ein Test-Behältnisinspektions-Modell.

Dabei erfolgt eine Bewertung des Test-Behältnisinspektions-Modells auf Grundlage einer Ausleitrate basierend auf der abgerufenen Vielzahl von ortsaufgelösten Sensordaten.

Eine Bewertung auf Grundlage dessen, ob ein zu erfassten Sensordaten zugehöriges Behältnis aus dem Behältnisteil-Strom auszuleiten ist oder nicht hängt wesentlich von einer vorgegebenen Schwellengröße ab, welche angibt, ab wann ein Behältnis mit einer Ausprägung eines Defekts auszuleiten ist oder nicht. Behältnisse, welche zwar nicht vollständig defektfrei sind, deren Defektausprägung allerdings nicht hinreichend ausgeprägt ist, dass das Behältnis als auszuleiten beurteilt wird, wird bei einer solchen Auswertung als "defektfrei" eingestuft.

Dabei bleiben - wie die Anmeldering im Rahmen der Erfindung erkannt hat - wertvolle Informationen unberücksichtigt, welche sich aus nicht vollständig defektfreien Behältnissen, die nicht ausgeleitet werden, ergeben können.

Der vorliegenden Erfindung liegt die Aufgabe zugrunde, die aus dem Stand der Technik bekannten Nachteile zu überwinden und ein Verfahren zum Betreiben einer Behältnisbehandlungsanlage zur Behandlung einer Vielzahl von Behältnisteilen für Behältnisse, eine Behältnisbehandlungsanlage sowie eine Steuervorrichtung für eine Behältnisbehandlungsanlage bereitzustellen, welche einen verbesserten, fein einstellbaren Betrieb mit einer hochqualitativen Behandlung der Behältnisteile bieten.

Die Aufgabe wird erfindungsgemäß durch die Gegenstände der unabhängigen Ansprüche gelöst. Vorteilhafte Ausführungsformen und Weiterbildungen der Erfindung sind Gegenstand der Unteransprüche.

Bei einem erfindungsgemäßen Verfahren zum Betreiben einer Behältnisbehandlungsanlage zur Behandlung einer Vielzahl von Behältnisteilen für Behältnisse und bevorzugt für Kunststoffbehältnisse und/oder Flaschen, transportiert eine Transporteinrichtung die Vielzahl von Behältnisteilen als Behältnisteil-Strom entlang eines vorgegebenen Transportpfads, bevorzugt von und/oder zu wenigstens einer Behandlungseinrichtung der Behältnisbehandlungsanlage und besonders bevorzugt von wenigstens einer Behandlungseinrichtung der Behältnisbehandlungsanlage zu wenigstens einer weiteren Behandlungseinrichtung der Behältnisbehandlungsanlage (zur Vornahme wenigstens eines Behandlungsschritts an der Vielzahl von Behältnisteilen).

Bei den Behältnissen handelt es sich bevorzugt um Kunststoffbehältnisse (insbesondere PET-Behältnisse), Behältnisse deren Hauptbestandteil aus Pulpe besteht und/oder Glasbehältnisse und/oder Dosen. Bei den Behältnissen kann es sich dabei um Behältnisse aus der Getränke- und/oder Lebensmittel- und/oder Kosmetikindustrie und/oder Pharmaindustrie handeln. Beispielsweise kann es sich um Dosen oder Flaschen, wie etwa Glasflaschen, Pulpeflaschen und Kunststoffflaschen handeln.

Unter einem "Behältnisteil für ein Behältnis" kann auch das Behältnis selbst verstanden werden. So könnte es sich bei dem Behältnisteil etwa um einen Vorformling handeln, aus welchem durch einen Umformvorgang das fertig ausgeformte Behältnis hergestellt wird, oder um das bereits fertig ausgeformte Behältnis handeln.

Bei dem Behältnisteil für ein Behältnis kann es sich auch um eine Ausstattung des Behältnisses, wie ein (bevorzugt wiederverschließbarer) Behältnisverschluss (etwa Drehverschluss oder Kappe), ein (PET- und/oder Kunststoff-)Deckel, ein Etikett, eine (Laser- oder Direkt-druck-)Markierung, ein Füllgut und/oder eine Verpackung des (fertigen) Behältnisses, eine Behältniszusammenstellung, oder dergleichen (sowie Kombinationen hiervon) handeln.

So kann es sich beispielsweise bei der Transporteinrichtung um eine Zuführung (etwa eine Zuführschiene, für Behältnisverschlüsse handeln, welche die Behältnisverschlüsse ausgehend von einer Sammeleinrichtung einer Verschließeinrichtung zum Verschließen von Behältnissen zuführt.

Bevorzugt handelt es sich bei dem Behältnisteil um einen von der Transporteinrichtung als (genau eine) Einheit transportablen und (unabhängig von weiteren transportierten Behältnisteilen) transportierten Gegenstand.

Bevorzugt führt die wenigstens eine (erste) Behandlungseinrichtung und/oder die (wenigstens eine) weitere Behandlungseinrichtung (insbesondere jeweils) wenigstens einen Behandlungsschritt an dem Behältnisteil (und bevorzugt an dem Behältnis) und/oder an der Vielzahl von Behältnisteilen durch.

Dabei wird der wenigstens eine Behandlungsschritt insbesondere an jedem Behältnisteil der Vielzahl von Behältnisteilen vorgenommen.

Der Behandlungsschritt der wenigstens einen (ersten) Behandlungseinrichtung und/oder der (wenigstens einen) weiteren Behandlungseinrichtung kann ausgewählt sein aus einer Gruppe von Behandlungsschritten, welche einen Spritzgießvorgang zum Herstellen eines Spritzgießteils (etwa Kunststoffvorformling), einen Reinigungsvorgang, einen Zerkleinerungsvorgang und/oder Zerteilungsvorgang (etwa im Rahmen eines Recyclingvorgangs), einen Umformvorgang (insbesondere ein (Streck-)Blasformen), einen (Laser-)Markierungsvorgang, einen Individualisierungsvorgang (etwa Anbringen eines QR-Codes), einen Ausleitvorgang (in welchem das Behältnisteil aus dem Behältnisteil-Strom der Behältnisbehandlungsanlage ausgeleitet wird), einen Sortiervorgang (in welchem die Behältnisteile sortenabhängig sortiert werden), einen Füllvorgang, einen Verschließvorgang, einen (insbesondere Direkt-)Druckvorgang, einen Etikettiervorgang, ein Vornehmen einer Laserdekoraktion, einen Vorgang zum Umreifen (eines Behältnisteils und bevorzugt eines Behältnisses), einen Verpackungsvorgang (insbesondere ein Abringen einer Primär- und/oder Sekundärverpackung, etwa Einschweißen mehrerer Behältnisse als Paket und/oder als Gebindeeinheit), insbesondere ein Vornehmen einer Schrumpfverpackung, eine Bestimmung der Zusammensetzung von Stoffen oder Stoffgemischen, etwa der Atmosphäre und/oder Luft in oder an einem Behältnisteil und bevorzugt Behältnis, beispielsweise mittels eines Massenspektrometers und/oder einer Geruchssensoreinrichtung, und dergleichen sowie Kombinationen hiervon umfasst.

Zur Durchführung (wenigstens) einer Behältnisinspektionsaufgabe erfasst wenigstens eine Sensoreinrichtung, insbesondere ortsaufgelöste, Sensordaten und bevorzugt Kamerabilder in Bezug auf die (zu inspizierenden und/oder transportierten) Behältnisteile, bevorzugt optisch, (bevorzugt während einem Arbeitsbetrieb der Behältnisbehandlungsanlage). Bei der Sensoreinrichtung handelt es sich bevorzugt um eine Sensoreinrichtung der Behältnisbehandlungsanlage und/oder einer nachfolgend näher beschriebenen Behältnisinspektionsvorrichtung.

Bevorzugt transportiert die Transporteinrichtung die Vielzahl (zu behandelnder und/oder behandelter) Behältnisteile (zu dessen Inspektion, um die Behältnisinspektionsaufgabe zu erfüllen) zu der Sensoreinrichtung, welche jeweils, insbesondere ortsaufgelöste, Sensordaten in Bezug auf die transportierten Behältnisteile (insbesondere einzeln) erfasst. Die Sensoreinrichtung und/oder die die wenigstens eine Sensoreinrichtung umfassende (nachfolgend näher beschriebene) Behältnisinspektionsvorrichtung kann zwischen der Behandlungseinrichtung und der wenigstens einen weiteren Behandlungseinrichtung angeordnet sein.

Bevorzugt wird das (zu inspizierende) Behältnisteil wenigstens bereichsweise und bevorzugt dessen aus wenigstens einer Beobachtungsrichtung beobachtbarer bzw. sichtbarer Behältnisteilbereich in den Sensordaten abgebildet.

Denkbar ist, dass zu jedem zu inspizierenden und/oder zur Sensoreinrichtung transportierten Behältnisteil einzeln bzw. Behältnisteil-individuell Sensordaten erfasst bzw. erhoben werden (in jeweils einem eigenen Erfassungsschritt der Sensoreinrichtung).

Es kann genau eine Sensoreinrichtung vorgesehen sein, welche die zur Durchführung der Behältnisinspektionsaufgabe erforderlichen Sensordaten in Bezug auf die Behältnisteile erfasst. Denkbar ist aber auch, dass hierfür mehrere Sensoreinrichtungen vorgesehen ist, welche beispielsweise die Sensordaten aus mehreren Aufnahmerichtungen in Bezug auf das Behältnisteil erfassen und/oder welche in einem mehrbahnigen Transportbereich jeweils die auf verschiedenen Bahnen transportierten Behältnisteile erfassen.

Bei den Sensordaten handelt es sich bevorzugt um ortsaufgelöste Sensordaten, welche insbesondere eine zu erfassende Eigenschaft (etwa Farbwert und/oder Grauwert und/oder Helligkeitswert) eines Bereichs des Behältnisteils abbilden. Bevorzugt geben die ortsaufgelösten Sensordaten einen Sensordatenverlauf in Abhängigkeit von wenigstens einer räumlichen und/oder geometrischen Koordinate und bevorzugt in Abhängigkeit von wenigstens zwei räumlichen und/oder geometrischen Koordinaten an (oder sind angebbar).

Bei den Sensordaten kann es sich - wie etwa bei den von einer Kamera erfassten Sensordaten - etwa um einen Farbwert und/oder Grauwert und/oder Helligkeitswert handeln.

Bei den von einer LIDAR-Einrichtung erfassten Sensordaten kann es sich um RGB-Werte und/oder Intensitätswerte handeln, welche für jeden erfassten Datenpunkt zusammen mit bzw. in Abhängigkeit von dessen X-, Y- und Z-Positionswert erfasst und abgelegt werden.

Denkbar ist auch, dass es sich bei den Sensordaten um frequenzaufgelöste Sensordaten handelt.

So kann etwa je Sensordatenpunkt wenigstens ein Intensitätswert in Abhängigkeit einer Frequenz der von der Sensoreinrichtung erfassten Strahlung erfasst werden, so dass die Sensordaten einen Sensorwertverlauf in Abhängigkeit einer Frequenz angeben.

Denkbar ist auch, dass es sich bei den Sensordaten um Spektrometer-Sensordaten handelt, welche bevorzugt von einem Massenspektrometer, welches etwa als Geruchssensor verwendet wird, erzeugt werden. Denkbar ist, dass damit etwa eine Zusammensetzung eines Gases und/oder Luft und/oder Luftgemischs, etwa einer Atmosphäre in einem Behältnis analysiert wird. Hier können etwa die erfassten Sensordaten einen Intensitätswertverlauf in Abhängigkeit eines Masse-zu-Ladung-Verhältnisses von in der Atmosphäre enthaltenen Atomen und Molekülen angeben.

Bevorzugt handelt es sich bei der Erfassung der insbesondere ortsaufgelösten Sensordaten um eine optische Erfassung. Bevorzugt handelt es sich bei den ortsaufgelösten Sensordaten um Kamerabilder.

Bevorzugt werden die Sensordaten in Bezug auf die zu inspizierenden Behältnisteile während des Transports dieser jeweils zu erfassenden Behältnisteile mit unveränderter insbesondere nicht reduzierter Transportgeschwindigkeit erfasst, also während sich die Behältnisteile in Bewegung befinden. Zur Erfassung der Sensordaten werden mit anderen Worten die Behältnisteile nicht abgebremst und/oder angehalten. Dies bietet den Vorteil einer hohen Durchlaufgeschwindigkeit und Produktionsgeschwindigkeit der Behältnisbehandlungsanlage.

Zur optischen Erfassung der Sensordaten kann eine Beleuchtung der Behältnisteile und bevorzugt Behältnisse vorgesehen sein, etwa eine Auflicht- und/oder eine Durchlichtbeleuchtung.

Eine, insbesondere Prozessor-basierte, Echtzeit-Auswertungseinrichtung wertet (zur Durchführung der (wenigstens einen) Behältnisinspektionsaufgabe) die insbesondere ortsaufgelösten Sensordaten, insbesondere in Echtzeit, mittels eines Behältnisinspektions-Modells, (im Rahmen eines Computer-implementierten Verfahrensschritts) aus.

Bei dem Behältnisinspektions-Modell handelt es sich bevorzugt um ein Behältnisinspektions-Modell maschinellen Lernens, welches einen Satz Parameter umfasst, welche auf Werte eingestellt sind, die als Ergebnis eines maschinellen Lernverfahrens (bzw. eines Trainingsprozesses) gelernt wurden.

Die Echtzeit-Auswertungseinrichtung ist bevorzugt Teil der Behältnisbehandlungsanlage und/oder der (nachfolgend näher beschriebenen) Behältnisinspektionsvorrichtung.

Erfindungsgemäß ist (bevorzugt der Echtzeit-Auswertungseinrichtung) ein auf einem und bevorzugt dem (obigen) maschinellen Lernverfahren basierender Satz von Behältnisteil-Merkmalen vorgegeben und/oder wird vorgegeben.

Bevorzugt werden (bevorzugt durch die Echtzeit-Auswertungseinrichtung) die erfassten, insbesondere ortsaufgelösten, Sensordaten in Bezug auf eine, von der Behältnisinspektionsaufgabe verschiedene Anlagen-Inspektionsaufgabe (bzw. zur Durchführung einer, von der Behältnisinspektionsaufgabe verschiedenen Anlagen-Inspektionsaufgabe) basierend auf dem vorgegebenen Satz von Behältnisteil-Merkmalen ausgewertet. Bevorzugt wird in Abhängigkeit des Inspektionsergebnisses der durchgeführten Anlagen-Inspektionsaufgabe wenigstens eine Anlageninspektionsgröße ermittelt, welche zur (wenigstens teilweisen automatischen und bevorzugt vollautomatischen) Steuerung und/oder Regelung der Behältnisbehandlungsanlage bereitgestellt wird. Dabei kann es sein, dass zur Durchführung der Anlagen-Inspektionsaufgabe insbesondere erfasste Sensordaten ausgewählt werden, welche ein vorgegebenes Inspektionsergebnis bei der durchgeführten Behältnisinspektionsaufgabe erreicht haben (etwa, dass kein vorgegebener Defekt erkannt wurde oder dass die Sensordaten im Allgemeinen als defektfrei eingeordnet wurden).

Zusätzlich oder alternativ werden bevorzugt die erfassten, insbesondere ortsaufgelösten, Sensordaten (insbesondere in Bezug auf eine, von der Behältnisinspektionsaufgabe verschiedene Anlagen-Inspektionsaufgabe bzw. zur Durchführung einer von der Behältnisinspektionsaufgabe verschiedenen Anlagen-Inspektionsaufgabe) in Bezug auf vorgegebene und/oder vorgebbare Referenzdaten durch Ermittlung einer Ähnlichkeitsgröße, welche charakteristisch ist für eine Ähnlichkeit der Sensordaten zu den Referenzdaten, ausgewertet. Dabei wird die Ähnlichkeitsgröße oder eine hiervon abgeleitete Größe zur Steuerung und/oder Regelung der Behältnisbehandlungsanlage bereitgestellt. Dabei wird bevorzugt die Ähnlichkeitsgröße basierend auf einem vorgegebenen, besonders bevorzugt auf dem maschinellen Lernverfahren basierenden (obig beschriebenen) Satz von Behältnisteil-Merkmalen ermittelt. Dabei kann die Ähnlichkeitsgröße als Anlageninspektionsgröße dienen. Denkbar ist auch, dass die Anlageninspektionsgröße in Abhängigkeit der Ähnlichkeitsgröße und bevorzugt in Abhängigkeit einer Vielzahl von Ähnlichkeitsgrößen ermittelt wird.

Bevorzugt wird die Behältnisbehandlungsanlage in Abhängigkeit der wenigstens einen (ermittelten) Anlageninspektionsgröße und/oder in Abhängigkeit des Inspektionsergebnisses der durchgeführten Anlagen-Inspektionsaufgabe (und/oder in Abhängigkeit der Ähnlichkeitsgröße) (wenigstens teilweise automatisch und bevorzugt vollautomatisch) gesteuert und/oder geregelt. Dabei wird bevorzugt eine Durchführung wenigstens eines von der Behältnisbehandlungsanlage durchgeführten Behandlungsschritts und besonders bevorzugt des wenigstens einen (an der Vielzahl von Behältnisteilen durchzuführenden) Behandlungsschritts und/oder ein Betriebszustand der Behältnisbehandlungsanlage (in Abhängigkeit der wenigstens einen Anlageninspektionsgröße und/oder in Abhängigkeit des Inspektionsergebnisses der durchgeführten Anlagen-Inspektionsaufgabe und/oder in Abhängigkeit der Ähnlichkeitsgröße) gesteuert und/oder geregelt.

Mit anderen Worten wird durch eine erste bevorzugte Ausführungsform des Verfahrens vorgeschlagen, dass nicht nur in einem maschinellen Lernverfahren ein Behältnisinpektions-Modell zur Auswertung der Sensordaten erzeugt wird, in welchem beispielsweise die Sensordaten entsprechend darin erkannter Defekte und/oder erkannter Behältnisteil-Sorten kategorisiert werden können, sondern dass daneben Mittel in Form der im Rahmen eines und bevorzugt desselben maschinellen Lernverfahren erhaltenen Satz von Behältnisteil-Merkmalen bereitgestellt werden, mittels welchen die Sensordaten auch in Bezug auf eine Funktionsweise und Steuerung/Regelung der Behältnisbehandlungsanlage ausgewertet werden können.

Mit anderen Worten wird durch eine zweite bevorzugte Ausführungsform des Verfahrens vorgeschlagen, die Sensordaten nicht nur mittels eines Behältnisinspektions-Modells maschinellen Lernens zur Durchführung einer Behältnisinspektionsaufgabe (etwa zur Kategorisierung in den Sensordaten erkannter Defekte an den Behältnisteilen und/oder erkannte Behältnisteil-Sorten), sondern auch mittels einer Ähnlichkeitsanalyse zu vorgegebenen Referenz-daten auszuwerten.

Bevorzugt handelt es sich bei den Referenzdaten um, bevorzugt von einer Sensoreinrichtung, erfasste, insbesondere ortsaufgelöste, Sensordaten (in Bezug auf ein Behältnisteil), welche als Referenz, also als Referenz-Sensordaten verwendet werden (im Nachfolgenden auch als Referenz-Sensordaten bezeichnet). Bei der Sensoreinrichtung kann es sich dabei um diejenige Sensoreinrichtung handeln, welche die, insbesondere ortsaufgelösten, Sensordaten erfasst (in Bezug auf welche die Ablegegröße zu ermitteln ist bzw. ermittelt wird).

Zusätzlich oder alternativ kann es sich um eine von der die, insbesondere ortsaufgelöste, Sensordaten erfassende Sensoreinrichtung (der Behältnisbehandlungsanlage) verschiedene (etwa baugleiche) Sensoreinrichtung handeln. Beispielsweise könnte es sich bei der die Referenz-Sensordaten erfassenden Sensoreinrichtung um eine weitere Sensoreinrichtung derselben Behältnisbehandlungsanlage handeln.

Daneben könnten etwa auch von einer baugleichen Sensoreinrichtung einer verschiedenen Behältnisbehandlungsanlage erfasste Sensordaten als Referenz-Sensordaten verwendet werden. Dies bietet den Vorteil, dass etwa überprüft werden kann, ob in der verschiedenen Behältnisbehandlungsanlage entdeckte, sehr selten auftretende Defekte oder sich aus einer Störung in der Behältnisbehandlungsanlage ergebende Merkmale in dem behandelten Behältnisteil auch in der betrachteten Behältnisbehandlungsanlage auftreten.

Die Referenzdaten sind dabei bevorzugt in einer Speichereinrichtung der Behältnisbehandlungsanlage und besonders bevorzugt der Behältnisinspektionsvorrichtung, welche die Sensoreinrichtung umfasst, abgelegt.

Insbesondere werden die erfassten, insbesondere ortsaufgelösten, Sensordaten mit den Referenzdaten verglichen und aus dem Vergleichsergebnis wird die Ähnlichkeitsgröße ermittelt.

Dabei kann die erste bevorzugte Ausführungsform und die zweite bevorzugte Ausführungsform des Verfahrens unabhängig in einem Verfahren vorliegen. Die erste und zweite bevorzugte Ausführungsform des Verfahrens können auch beide in einem gemeinsamen Verfahren verwirklicht werden. Die nachfolgend aufgeführten Merkmale können sich daher sowohl auf die obig beschriebene erste bevorzugte als auch auf die zweite bevorzugte Ausführungsform oder aber auf eine Verwirklichung beider bevorzugter Ausführungsformen des Verfahrens beziehen.

Bevorzugt handelt es sich bei der Ähnlichkeitsgröße um eine nicht diskrete Größe, welche insbesondere nicht nur zwei oder eine endliche (fest vorgegebene) Anzahl an Werten annehmen kann. Bevorzugt handelt es sich bei der Ähnlichkeitsgröße um eine kontinuierliche Größe. Bevorzugt gibt die Ähnlichkeitsgröße einen Ähnlichkeitsgrad an.

Während bevorzugt das Behältnisinspektions-Modell den erfassten Sensordaten wenigstens eine Kategorie aus einer Vielzahl von vorgegebenen Kategorien zuordnet, können die Sensordaten hierdurch bevorzugt durch Ermittlung einer nicht-diskreten Ähnlichkeitsgröße noch feiner ausgewertet werden. Hierdurch können etwa langsam fortschreitende Entwicklungstrends, die sich etwa bei einer Alterung von Bauteilen der Behältnisbehandlungsanlagen oder einer Änderung der Produktionsmaterialien ergeben können und auf die Behandlung der Behältnisteile auswirken, bereits in einer sehr frühen Phase entdeckt werden, in welcher eine Kategorisierung der Behältnisteile durch die veränderte Behandlung des Behältnisteils noch nicht beeinflusst ist.

Bei einem bevorzugten Verfahren handelt es sich bei dem Satz von Behältnisteil-Merkmalen um einen, im Rahmen eines und bevorzugt des maschinellen Lernverfahrens, bevorzugt automatisch, erhaltenen Satz von, insbesondere von einem neuronalen Netz, extrahierten Behältnisteil-Merkmalen. Bevorzugt ergibt sich der Satz von Behältnisteil-Merkmalen aus dem maschinellen Lernverfahren (automatisch).

Dabei handelt es sich bei dem maschinellen Lernverfahren bevorzugt um das maschinelle Lernverfahren, welches zum Erhalt des Behältnisinspektions-Modells durchgeführt wurde. Denkbar ist aber auch, dass sich der Satz von Behältnisteil-Merkmalen aus einem maschinellen Lernverfahren in Bezug auf eine von der Behältnisinspektionsaufgabe verschiedenen (von der Echtzeit-Auswertungseinrichtung auf Grundlage der erfassten, insbesondere ortsaufgelösten, Sensordaten durchzuführenden) Trainings-Behältnisinspektionsaufgabe handelt.

Dabei ist bevorzugt die Trainings-Behältnisinspektionsaufgabe von einer Behältnisinspektionsaufgabe verschieden. Die Behältnisteil-Merkmale werden bevorzugt bei einem maschinellen Lernverfahren bzw. bei Vornahme eines maschinellen Lernverfahrens extrahiert, welches in Bezug auf eine Trainings-Behältnisinspektionsaufgabe durchgeführt wird. Mit der Durchführung des maschinellen Lernverfahrens soll ein (trainierter) Algorithmus bzw. ein (trainiertes) Modell (maschinellen Lernens) erhalten werden, welches zur Erfüllung bzw. zur Durchführung der Trainings-Behältnisinspektionsaufgabe dient.

Bevorzugt handelt es sich bei dem Satz von extrahierten Behältnisteil-Merkmalen um einen, im Rahmen des in Bezug auf die Trainings-Behältnisinspektionsaufgabe durchgeführten maschinellen Lernverfahrens (automatisch) extrahierten Satz von (extrahierten) Behältnisteil-Merkmalen.

Dass ein von der Behältnisinspektionsaufgabe verschiedene Trainings-Behältnisinspektionsaufgabe verwendet werden kann, bietet den Vorteil, dass zur Merkmalsextraktion lediglich ein einziger Trainingsprozess bzw. ein einziges maschinelles Lernverfahren, nämlich der im Rahmen zur Trainings-Behältnisinspektionsaufgabe durchgeführte Trainingsprozess bzw. maschinelle Lernverfahren, ausreichend ist. Die hier erhaltenen extrahierten Behältnisteil-Merkmale werden sodann für andere Behältnisinspektionsaufgaben verwendet.

Beispielsweise können die extrahierten Merkmalsvektoren einer Flasche in einem (Kamera- )Bild in mehreren Inspektionsaufgaben/ Klassifikationen verwendet werden.

Beispiel für zwei Inspektionsaufgaben/Klassifikationen:
1. Zeigen die Merkmalsvektoren eine braune Flasche?
2. Zeigen die Merkmalsvektoren eine verschlossene Flasche?

Beides ist in einem Bild und damit auch in dem extrahierten Merkmalsvektor erkennbar. Im ersten Fall wird der Algorithmus maschinellen Lernens, etwa kNN, dann mit Merkmalsvektoren von braunen und andersfarbigen Flaschen "trainiert". Im zweiten Fall mit Merkmalsvektoren von verschlossenen und unverschlossenen Flaschen.

Die Merkmale müssen nur einmal aus jedem Bild extrahiert werden.

Bei einem weiter bevorzugten Verfahren handelt es sich bei dem Lernverfahren, im Rahmen dessen der Satz von Behältnisteil-Merkmalen extrahiert wird, um ein überwachtes Lernverfahren. Bevorzugt wird zur Durchführung des überwachten Lernverfahrens ein Satz von Trainingsdaten verwendet, welcher (erfasste) Sensordaten in Bezug auf Behältnisteile umfasst, welche mit einem in dem jeweiligen Fall zu erhaltenden Inspektionsergebnis der vorgegebenen (Trainings-)Behältnisinspektionsaufgabe (etwa eine vorgegebene Vielzahl von Kategorien) gelabelt bzw. gekennzeichnet sind.

Bei einem weiter bevorzugten Verfahren handelt es sich bei dem überwachten Lernverfahren um einen K-nächste-Nachbarn-Algorithmus (englisch: "K nearest neighbour"-Algorithmus, auch abgekürzt als "k-NN" oder "KNN"). Bei diesem handelt es sich vorteilhaft um einen leicht an neu hinzugefügte Trainingsmuster anpassbaren und einfachen Algorithmus. Der K-nächste-Nachbarn-Algorithmus benötigt lediglich einen k-Wert und eine Distanzmetrik (Abstandsmetrik), was im Vergleich zu anderen Algorithmen im maschinellen Lernen wenig ist.

Bei einem weiter bevorzugten Verfahren wird (als Lernverfahren) ein traditioneller machine Learning Algorithmus (Maschinenlern-Algorithmus), wie beispielsweise Decision Trees (Decision Tree Learning, dt.: "Entscheidungsbaum-Lernen", wobei ein Entscheidungsbaum ein nichtparametrischer, überwachter Lernalgorithmus mit bevorzugt einer hierarchischen, baumartigen Struktur ist, der beispielsweise sowohl für Klassifizierungs- als auch für Regressionsaufgaben verwendet wird), Random Forests (deutsch: Zufallswald) oder Random Decision Forest, Logistic Regression (dt. logistische Regression), k-Means Clustering, Support Vector Machines (Abkürzung SVM, nicht-gebräuchliche dt. Übersetzung: "Stützvektormaschine" oder Stützvektormethode) verwendet.

Bei den extrahierten Behältnisteil-Merkmalen handelt es sich dabei insbesondere nicht um vorgegebene Merkmale, auch nicht um eine Auswahl (etwa von einem Nutzer) vorgegebener Merkmale. Bei den extrahierten Behältnisteil-Merkmalen handelt es sich insbesondere um abstrakte Merkmale, welche etwa einen Hell-Dunkel-Kontrast, eine für eine Häufigkeit von geraden Linien charakteristische Größe (etwa wie viele gerade Linien), eine Helligkeit oder einen Helligkeitsverlauf, Formen von Kontur(-Linien) und/oder Begrenzungslinien, Ecken, Formen, Anzahlen von Ecken, Krümmungen, Kombinationen hiervon und dergleichen angeben oder hierfür charakteristisch sind.

Bevorzugt sind die (insbesondere alle) Behältnisteil-Merkmale automatisch, bevorzugt im Rahmen des maschinellen Lernverfahrens, erzeugt (und insbesondere nicht ausgewählt).

Bevorzugt wird der Satz extrahierter Behältnisteil-Merkmale nicht angepasst und/oder verändert, auch nicht bei einer Festlegung einer (neuen und/oder weiteren und/oder anzupassenden) Behältnisinspektionsaufgabe und/oder einer neuen/geänderten Anlagen-Inspektionsaufgabe und/oder bei dem Vorgeben neuer/geänderter Referenzdaten bzw. Referenz-Sensordaten.

Bevorzugt kann auf den Satz von Behältnisteil-Merkmalen zugegriffen werden, insbesondere auch auf die einzelnen Behältnisteil-Merkmale des Satzes von Behältnisteil-Merkmalen. Der Satz von Behältnisteil-Merkmalen ist also nicht implizit in einem Algorithmus zur Bildauswertung (quasi als "Black Box") intrinsisch enthalten, sondern ist so abgelegt, dass auf diesen eigenständig und separat zugegriffen werden kann. Besonders bevorzugt kann der Satz von Behältnisteil-Merkmalen auch (insbesondere unabhängig von weiteren Software-Bausteinen) separat ausgetauscht werden. Denkbar ist auch, dass dieser Satz von Behältnisteil-Merkmalen nur für sich genommen ausgegeben und/oder übermittelt werden kann.

Denkbar ist, dass eine Anzahl der Behältnisteil-Merkmale des Satzes extrahierter bzw. zu extrahierenden Behältnisteil-Merkmalen, etwa von einem Nutzer der Behältnisbehandlungsanlage, vorgegeben wird und/oder ist. Bevorzugt wird die vorgegebene Anzahl der Behältnisteil-Merkmale bei der Ermittlung des Satzes zu extrahierender Behältnisteil-Merkmale berücksichtigt. Diese vorgegebene Anzahl der Behältnisteil-Merkmale kann etwa von dem Nutzer der Behältnisbehandlungsanlage an einen externen Server, welcher die Ermittlung des Satzes zu extrahierender Behältnisteil-Merkmale durchführt, übermittelt werden. Denkbar ist aber auch, dass die Anzahl der Behältnisteil-Merkmale von einem Hersteller der Behältnisbehandlungsanlage vorgegeben ist/wird und insbesondere durch die Behältnisbehandlungsanlage (oder von einem Bediener dieser) nicht beeinflussbar ist.

Bei einem weiter bevorzugten Verfahren ist bzw. wird durch den Satz von extrahierten Behältnisteil-Merkmalen ein Merkmalsraum aufgespannt bzw. es wird ein durch den Satz von Behältnisteil-Merkmalen aufgespannter Merkmalsraum bereitgestellt. Mit anderen Worten kann ein Merkmalsraum gebildet werden, welcher durch den bereitgestellten Satz von extrahierten Behältnisteil-Merkmalen aufgespannt wird/ist.

Bevorzugt wird eine Distanzmetrik (Abstandsmetrik) in Bezug auf den Merkmalsraum bereitgestellt.

Bevorzugt wird die Ähnlichkeitsgröße mittels der Distanzmetrik (Abstandsmetrik) ermittelt. Dabei werden bevorzugt die, insbesondere ortsaufgelösten, Sensordaten und/oder die Referenz-Sensordaten jeweils in dem Merkmalsraum (als Merkmals-Vektor) dargestellt. Ein Abstand zwischen diesen beiden Merkmalsvektoren wird bevorzugt mittels der Distanzmetrik (Abstandsmetrik) ermittelt. Dieser Abstand oder eine hierfür charakteristische Größe wird bevorzugt als Ähnlichkeitsgröße zwischen den erfassten, insbesondere ortsaufgelösten, Sensordaten und den Referenz-Sensordaten verwendet.

Bei den Referenzdaten könnte es sich beispielsweise bereits um eine Darstellung von (Referenz-)Sensordaten im Merkmalsraum handeln. Die Referenzdaten könnten also beispielsweise bereits ein Merkmals-Vektor sein (oder hierfür charakteristisch sein). Bereits als Merkmals-Vektor angegebene (vorgegebene) Referenzdaten bieten den Vorteil einer deutlich geringeren Datengröße.

Mit anderen Worten wird bevorzugt eine Distanzmetrik (Abstandsmetrik) in Bezug auf den Merkmalsraum bereitgestellt, wobei zusätzlich oder alternativ (bevorzugt die Echtzeit-Auswertungseinrichtung) die Distanzmetrik (Abstandsmetrik) als Ähnlichkeitsmaß zwischen, insbesondere ortsaufgelösten, Sensordaten verschiedener Behältnisteile und bevorzugt verschiedener Behältnisse und/oder als Ähnlichkeitsmaß zwischen erfassten Sensordaten und Referenz-Sensordaten verwendet.

Bevorzugt wird (im Rahmen der Durchführung der Anlagen-Inspektionsaufgabe) eine Ähnlichkeit zwischen erfassten, insbesondere ortsaufgelösten, Sensordaten eines Behältnisteils mit denjenigen eines anderen Behältnisteils (etwa als Referenz-Sensordaten vorgegeben) mittels der Distanzmetrik beurteilt.

Bevorzugt wird zu allen erfassten (insbesondere ortsaufgelösten) Sensordaten, also beispielsweise für jedes aufgenommene Kamerabild, basierend auf dem Satz von extrahierten Behältnisteil-Merkmalen ein Merkmalsvektor erstellt. Bei dem Merkmalsvektor kann es sich beispielsweise um einen 256-dimensionalen Vektor handeln.

Bevorzugt weist der Merkmalsvektor höchstens 512 Dimensionen, bevorzugt höchstens 256 Dimensionen und besonders bevorzugt höchstens 128 Dimensionen auf. Bevorzugt weist der Merkmalsvektor mindestens 16, bevorzugt mindestens 32, bevorzugt mindestens 64 und besonders bevorzugt mindestens 128 Dimensionen auf. Grundsätzlich könnten aber auch Merkmalsvektoren mit mehr als 512 Dimensionen bzw. ein Merkmalsraum entsprechend höherer Dimensionalität verwendet werden.

Bevorzugt kann mittels der Distanzmetrik der Abstand des Merkmalsvektors von in Bezug auf ein erstes Behältnisteil erfassten Sensordaten und dem Merkmalsvektor von den in Bezug auf ein weiteres Behältnisteil erfassten Sensordaten ermittelt werden.

Mit anderen Worten wird bevorzugt die Distanzmetrik als Ähnlichkeitsmaß zur Beurteilung der Ähnlichkeit zwischen den erfassten, insbesondere ortsaufgelösten, Sensordaten und den Referenzdaten, insbesondere den Referenz-Sensordaten, zur Ermittlung der Ähnlichkeitsgröße verwendet.

Bei einem weiter bevorzugten Verfahren wird als Distanzmetrik eine euklidische Metrik und/oder eine Kosinus-Ähnlichkeit in dem Merkmalsraum verwendet. Die Kosinus-Ähnlichkeit (im engl. auch bekannt als "Cosine Similarity" oder "Cosine Distance") ist ein Maß für die Ähnlichkeit zweier Vektoren, bei welcher der Kosinus des Winkels zwischen beiden Vektoren bestimmt wird. Die Kosinus-Ähnlichkeit kann insbesondere als Maß verstanden werden, wie ausgeprägt zwei Vektoren in die gleiche Richtung zeigen. Die Kosinus-Ähnlichkeit zwischen zwei Vektoren a und b kann insbesondere berechnet werden aus dem Standardskalarprodukt aus den Vektoren a und b, dividiert durch die euklidische Norm aus a und die euklidische Norm aus b, also: Kosinus-Ähnlichkeit = (**a** · **b**)/(∥**a**∥ ∥**b**∥).

Ein mit der Distanzmetrik erhaltener (vergleichsweise) geringer Abstand zwischen zwei Merkmalsvektoren (im Merkmalsraum) wird bevorzugt als geringe Ähnlichkeit zwischen den beiden, den jeweiligen Merkmalsvektoren entsprechenden Sensordaten (bzw. Sensordaten und Referenz(Sensor-)Daten) angesehen. Umgekehrt wird bevorzugt ein mit der Distanzmetrik erhaltener (vergleichsweise) großer Abstand zwischen zwei Merkmalsvektoren (im Merkmalsraum) als hohe Ähnlichkeit zwischen den beiden, den jeweiligen Merkmalsvektoren entsprechenden Sensordaten (bzw. Sensordaten und Referenz(-Sensor-)Daten) angesehen.

Bei einem weiter bevorzugten Verfahren wird auf Grundlage der, auf dem Satz von Behältnisteil-Merkmalen basierenden Auswertung der in Bezug auf ein Behältnisteil erfassten Sensordaten eine wenigstens abschnittsweise Nachverfolgung einer Bewegung des Behältnisteils durch die Behältnisbehandlungsanlage vorgenommen. Dies bietet den Vorteil, dass festgestellt werden kann, ob das Behältnisteil bereits die Sensoreinrichtung oder mehrere Sensoreinrichtungen der Behältnisbehandlungsanlagen erreicht/passiert hat und/oder ob ein inspiziertes Behältnisteil bereits zum wiederholten Male die Sensoreinrichtung erreicht hat.

Bei einem weiter bevorzugten Verfahren wird die Nachverfolgung des Behältnisteils vorgenommen, ohne dass ein zur Individualisierung vorgenommener und/oder vorgesehener Behandlungsschritt an dem Behältnisteil vorgenommen wurde und/oder ist. So wurde etwa das Behältnisteil nicht mit einem das Behältnisteil individualisierenden eineindeutigen Code, etwa als QR-Code ausgestaltet, oder dergleichen versehen. Die Anmelderin hat herausgefunden, dass einzig auf Grundlage der verschiedenartigen Ausprägungen von (in einem maschinellen Lernverfahren extrahierten) Behältnisteil-Merkmalen, Behältnisteile identifiziert und wiedererkannt werden können.

So kann etwa die Sensoreinrichtung (bzw. die Echtzeit-Auswertungseinrichtung) eine Verunreinigung eines verunreinigten Behältnisses erkennen und das Behältnis als Reaktion dieser Behältnisinspektionsaufgabe (hier der Erkennung einer Verunreinigung) einer Reinigungseinrichtung zum Reinigen des Behältnisses zuführen. Nach absolviertem Reinigungsvorgang kann das Behältnis mittels der Transportvorrichtung erneut der Behältnisinpektionsvorrichtung und insbesondere der Sensoreinrichtung zugeführt werden. Basierend auf der Auswertung der nun in Bezug auf das Behältnis erfassten Sensordaten nach einem durchlaufenen Reinigungsvorgang kann vorteilhaft nicht nur erkannt werden, dass die Verunreinigung des Behältnisses (weiterhin) besteht, sondern auch, dass dieses Behältnis (mit dieser Verunreinigung) bereits einmal von der Sensoreinrichtung erfasst wurde und daher bereits (wenigstens einmal) einen Reinigungsvorgang durchlaufen hat. So kann etwa verhindert werden, dass etwa nicht von der Reinigungseinrichtung behebbare Verunreinigungen dazu führen, dass dasselbe Behältnis einen Reinigungs-Inspektions-Zyklus mehrmals durchläuft bzw. durchlaufen hat und damit Kapazitäten für andere Behältnisse blockiert.

Bevorzugt wird gezählt (insbesondere durch die Echtzeit-Auswertungseinrichtung und/oder die Behältnisinspektionsvorrichtung), wie oft ein Behältnisteil bereits von der Sensoreinrichtung erfasst wurde bzw. diese erreicht hat. Bevorzugt ist eine Maximalzahl (von einem Bediener) vorgebbar und/oder vorgegeben (und in einer Speichereinrichtung der Behältnisinspektionsvorrichtung und/oder der Behältnisbehandlungsanlage abgelegt und/oder ablegbar), und bevorzugt wird eine Steuerung und/oder Regelung der Behältnisbehandlungsanlage, und insbesondere einer Ausleiteinrichtung zur Ausleitung von, von der Sensoreinrichtung inspizierter Behältnisteile, in Abhängigkeit der vorgegebenen Maximalzahl im Vergleich zur Zahl, wie oft das jeweilige Behältnisteil die Sensoreinrichtung bereits erreicht hat und/oder erfasst wurde.

Bei einem weiter bevorzugten Verfahren werden basierend auf dem Satz von Behältnisteil-Merkmalen die in Bezug auf ein Behältnisteil erfassten Sensordaten dahingehend ausgewertet, dass wenigstens eine für das Behältnisteil charakteristische Identifikationsgröße ermittelt wird.

Bevorzugt wird als Identifikationsgröße ein Merkmalsvektor verwendet, mittels dem die zugehörigen erfassten Sensordaten in dem Merkmalsraum darstellbar sind bzw. dargestellt werden. Diese Identifikationsgröße wird bevorzugt auf einer Speichereinrichtung (insbesondere der Behältnisinspektionsvorrichtung und/oder der Echtzeit-Auswertungseinrichtung und/oder der Behältnisbehandlungsanlage) abgelegt. Dies bietet den Vorteil, dass auf ein Ablegen der Sensordaten verzichtet werden kann, wodurch Speicherplatz eingespart werden kann.

Bevorzugt wird diese Identifikationsgröße mit (wenigstens) einer zu weiteren, von der Sensoreinrichtung erfassten Sensordaten ermittelten Identifikationsgröße verglichen. Bevorzugt wird ein Abstand zwischen den beiden als Identifikationsgröße verwendeten Merkmalsvektoren ermittelt. Bevorzugt wird auf Grundlage des (mittels der Distanzmetrik) ermittelten Abstands beurteilt, ob es sich bei den jeweils zugehörigen erfassten Sensordaten um Sensordaten desselben Behältnisteils handelt.

Bevorzugt ist hierfür ein Schwellenwert-Abstand vorgegeben und/oder (von einem Bediener) vorgebbar. Unterschreitet die (mittels der Distanzmetrik ermittelte) Distanz (Abstand) zwischen zwei Merkmalsvektoren (bzw. Identifikationsgrößen) diesen Schwellenwert-Abstand, wird als Beurteilungsergebnis angenommen, dass es sich bei den zugehörigen erfassten Sensordaten um Sensordaten desselben Behältnisteils handelt.

Bei einem weiter bevorzugten Verfahren wird auf Grundlage der Auswertung der in Bezug auf ein Behältnisteil erfassten Sensordaten mittels dem Satz von Behältnisteil-Merkmalen überprüft, ob ein individuelles Behältnisteil (bevorzugt wiederholt) die Sensoreinrichtung oder wenigstens eine weitere Sensoreinrichtung der Behältnisbehandlungsanlage erreicht hat. Bevorzugt wird hierfür eine Ähnlichkeitsgröße ermittelt, welche charakteristisch ist für die Ähnlichkeit der jeweils erfassten Sensordaten. Die Ähnlichkeitsgröße kann dabei etwa mit obig beschriebener Distanzmetrik (als Maß für die Ähnlichkeit) ermittelt werden.

Dabei ist die wenigstens eine weitere Sensoreinrichtung beispielsweise stromabwärts in Bezug auf die Transportrichtung der Behältnisteile angeordnet. Bevorzugt erfasst neben der Sensoreinrichtung auch die wenigstens eine weitere Sensoreinrichtung Sensordaten in Bezug auf das (bevorzugt jedes) Behältnisteil. Bevorzugt erfasst die Sensoreinrichtungen das Behältnisteil aus derselben Erfassungsrichtung in Bezug auf das Behältnisteil. Bevorzugt kann durch eine Ermittlung der (wie obig vorgeschlagenen) Ähnlichkeitsgröße basierend auf der Distanzmetrik auf Grundlage der jeweils erfassten Sensordaten beurteilt werden, ob es sich um dasselbe Behältnisteil handelt. So kann festgestellt werden, ob und/oder wann das Behältnisteil die wenigstens eine weitere Sensoreinrichtung erreicht. Ist eine Vielzahl weiterer Sensoreinrichtungen (etwa entlang des gesamten Transportpfads durch die Behältnisbehandlungsanlage) vorgesehen, kann eine Bewegung des (individuellen) Behältnisteils durch die (gesamte) Behältnisbehandlungsanlage nachverfolgt werden.

Bei einem weiter bevorzugten Verfahren wird zur Überprüfung einer wiederholten Erreichung der das individuelle Behältnisteils erfassenden Sensoreinrichtung basierend auf dem Satz von Behältnisteil-Merkmalen eine für das Behältnisteil charakteristische Identifikationsgröße (etwa ein Merkmalsvektor) ermittelt und auf Grundlage der ermittelten Identifikationsgröße wird eine Ähnlichkeit zu nachfolgend von der Sensoreinrichtung erfassten Sensordaten ermittelt. Beispielsweise kann die Ähnlichkeit ermittelt werden, indem die Distanz zu der als Merkmalsvektor gewählten Identifikationsgröße und einem Merkmalsvektor, der zu den nachfolgend von der Sensoreinrichtung erfassten Sensordaten ermittelt wird, bestimmt wird.

Bei einem weiter bevorzugten Verfahren wird bei festgestellter wiederholter Erreichung der Sensoreinrichtung durch ein Behältnisteil ein erneuter Transport des Behältnisteils zu dieser Sensoreinrichtung, bevorzugt durch individuelle Änderung wenigstens eines bisherigen Behandlungsschrittes in Bezug auf das Behältnisteil und besonders bevorzugt durch Ausschleusen des Behältnisteils aus dem Behältnisteil-Strom, verhindert. So kann etwa beispielsweise ein Reinigungsschritt abgeändert werden, den dieses Behältnisteil zu durchlaufen hat und/oder das Behältnisteil kann (endgültig) aus dem Behältnisteil-Strom ausgeschleust werden.

Bei einem weiter bevorzugten Verfahren sind (nicht nur in der obig erwähnten zweiten bevorzugten Ausführungsform sondern auch in der ersten bevorzugten Ausführungsform des vorgeschlagenen Verfahrens) Referenz-Daten vorgegeben und/oder vorgebbar und die Steuerung und/oder Regelung basiert auf einer ermittelten Ähnlichkeitsgröße, welche charakteristisch ist für eine Ähnlichkeit zwischen den Referenzdaten und (in Bezug auf wenigstens ein Behältnisteil und bevorzugt in Bezug auf eine Vielzahl von Behältnisteilen jeweils) erfassten Sensordaten.

Bei einem weiter bevorzugten Verfahren wird wenigstens ein Behandlungsschritt und/oder wenigstens ein Betriebszustand der Behältnisbehandlungsanlage in Abhängigkeit des Inspektionsergebnisses der Anlagen-Inspektionsaufgabe geändert und/oder angepasst. Bevorzugt wird der Behandlungsschritt und/oder der wenigstens eine Betriebszustand in Abhängigkeit des Inspektionsergebnisses der Anlagen-Inspektionsaufgabe, und besonders bevorzugt in Abhängigkeit einer auf Grundlage der erfassten Sensordaten und (insbesondere vorgegebenen) Referenz-(Sensor-)Daten, ermittelten Ähnlichkeitsgröße, gesteuert und/oder geregelt.

Beispielsweise sind/werden als Referenzdaten (erfasste) Sensordaten (als Referenz-Sensordaten) oder Merkmalsvektoren zu Behältnisteilen vorgegeben, welche (bevorzugt mittels des Behältnisinspektions-Modells maschinellen Lernens) derart klassifiziert werden/sind, dass das zugehörige Behältnisteil nicht defektfrei und/oder auszuleiten ist. So könnte das in den Referenz-Sensordaten abgebildete Behältnisteil bzw. das dem Merkmalsvektor zugehörige Behältnisteil einen vorgegebenen Defekt aufweisen, welcher bei Durchführung der Behältnisinspektionsaufgabe von der Echtzeit-Auswertungseinrichtung als solcher erkannt wird (etwa mittels des Behältnisinspektions-Modells maschinellen Lernens).

Mittels dieser Referenzdaten werden bevorzugt weitere von der Sensoreinrichtung erfasste Sensordaten, welche bei der Durchführung der Behältnisinspektionsaufgabe als defektfrei (zumindest in Bezug auf den vorgegebenen Defekt) erkannt wurden/sind, daraufhin untersucht, ob diese (dennoch) eine (etwa von einem Bediener, vorgegebene Mindest-)Ähnlichkeit zu diesen (Defekt-behafteten) Referenzdaten aufweisen und/oder welchen Ähnlichkeitsgrad diese zu diesen Referenzdaten aufweisen. Wie obig erläutert kann eine Ermittlung einer Ähnlichkeit bzw. eines Ähnlichkeitsgrads der jeweiligen Sensordaten zu den Referenzdaten erfolgen, indem mittels der Distanzmetrik ein Abstand zwischen einer Darstellung der Sensordaten im Merkmalsraum und einer Darstellung der Referenzdaten im Merkmalsraum (jeweils als Merkmalsvektor) berechnet wird.

Liegt der berechnete Abstand unter einem vorgegebenen Wert (welcher die Mindest-Ähnlichkeit angibt), wird das zugehörige Behältnisteil als ausreichend ähnlich (in Bezug auf die vorgegebene Mindest-Ähnlichkeit) zu den Referenzdaten angesehen. In diesem Falle kann es sich beispielsweise um ein Behältnisteil handeln, welches bereits geringe Ausprägungen des vorgegebenen Defekts aufweist, das Behältnisteil aber (noch) die (erforderlichen, etwa von dem Bediener vorgegebenen) Qualitätsanforderungen erfüllt.

Beispielsweise könnte eine Mehrweg-Glasflasche (als Behältnisteil) bereits ein sogenanntes "Scuffing" an der äußeren Glasoberfläche zeigen, wobei das Scuffing - ein Abrieb der Glasoberfläche beispielsweise bei wiederholtem Führen durch eine Mehrweg-Glasflaschen-Füllanlage entstehen kann. Kleinere Ausprägungen des "Scuffing" sind zwar optisch erkennbar, führen aber zu keinerlei Qualitätsminderung. Erst bei sehr deutlichen Ausprägungen des Scuffings wird dieses Glasbehältnis aus dem Behältnis-Strom entfernt und etwa einer Recycling-Anlage zugeführt.

Mittels der Durchführung der Behältnisinspektionsaufgabe werden Behältnisteile (hier Glasflaschen) erkannt, welche aufgrund einer erkannten sehr deutlichen "Scuffing"-Ausprägung aus dem Behältnis-Strom zu entfernen und der Recyclinganlage zuzuführen sind. Vorgeschlagen wird nun, mittels der Durchführung der Anlagen-Inspektionsaufgabe durch einen zusätzlichen Abgleich der jeweils zu den (allen) inspizierten Behältnisteilen erfassten Sensordaten mit Referenzdaten bereits eine Information darüber zu erhalten, ob und wieviele nicht ausgeleitete und/oder nicht auszuleitende Behältnisteile (hier Glasflaschen) in dem von der Sensoreinrichtung inspizierten Behältnisteil-Strom vorhanden sind, welche bereits Scuffing zu einem vorgegebenen Ausprägungsgrad zeigen. Dies kann etwa eine Information über die in der Behältnisbehandlungsanlage behandelte Vielzahl von Behältnisteilen, etwa dessen Alter und/oder Zustand, angeben.

Weiter bevorzugt wird basierend auf derart erkanntem Scuffing in geringer Ausprägung, also (allgemein) basierend auf ermittelten Ähnlichkeitsgrößen bevorzugt einer Vielzahl erfasster Sensordaten jeweils in Bezug auf die vorgegebenen Referenzdaten, eine Einstellung an der Behältnisbehandlungsanlage vorgenommen, etwa eine Steuerung einer Transportgeschwindigkeit und/oder einer Änderung einer Führungseinrichtung zum Führen der zu transportierenden Behältnisse.

Als Beispiel eines Defekts wurde obig das sogenannte "Scuffing" vorgestellt.

Zusätzlich oder alternativ sind/werden bevorzugt Referenzdaten vorgegeben, welche Behältnisteile mit einem vorgegebenen Defekt abbilden, welcher sich bei Vornahme wenigstens eines bzw. des wenigstens einen Behandlungsschritts an dem Behältnisteil ergeben kann. Auf diese Weise ist es vorteilhaft möglich, auf Störungen und/oder nachteilige Entwicklungen im Behandlungsablauf der Behältnisbehandlungsanlage, beispielsweise im Blasformprozess, zu schließen.

Dabei kann die Ursache des vorgegebenen Defekts bei der Durchführung des Behandlungsschritts selbst und/oder bei einem Zustand der Behältnisbehandlungsanlage (oder der Behältnisbehandlungseinrichtung), wie etwa eine vorliegende Prozesstemperatur, wie eine Temperatur in einer Heizeinrichtung der Kunststoffvorformlinge) liegen.

Derartige Referenzdaten bzw. Referenz-Sensordaten können dabei von vorherigen Betrieben der Behältnisbehandlungsanlage oder von anderen (insbesondere baugleichen) Behältnisbehandlungsanlagen stammen.

Bevorzugt werden (wie obig beschrieben) von der Sensoreinrichtung erfasste Sensordaten (im Rahmen der Durchführung der Anlagen-Inspektionsaufgabe) in Bezug auf die vorgegebenen Referenzdaten ausgewertet, indem eine (etwa wie obig erläuterte) Ähnlichkeitsgröße ermittelt wird (bevorzugt ein Abstand mittels einer Distanzmetrik).

Bevorzugt wird diese Anlagen-Inspektionsaufgabe in Bezug auf eine Vielzahl von (in Bezug auf die Behältnisteile) erfassten Sensordaten durchgeführt, bei welchen bei der Durchführung der Behältnisinspektionsaufgabe der vorgegebene Defekt nicht erkannt wurde, und/oder deren zugeordnete Behältnisteile nicht auf Grundlage des Inspektionsergebnisses der Behältnisinspektionsaufgabe aus dem Behältnisteil-Strom auszuleiten sind/ausgeleitet wurden. Dies bietet den Vorteil, dass gerade diejenigen Sensordaten näher analysiert werden, deren Qualität als ausreichend beurteilt wird. Auf diese Weise können gerade sich bereits leicht abzeichnende unerwünschte Ausgestaltungen der Behältnisteile entdeckt werden.

Bevorzugt wird auf Grundlage der zu einer Vielzahl von erfassten Sensordaten durchgeführten Anlagen-Inspektionsaufgaben und/der auf Grundlage der zu einer Vielzahl von erfassten Sensordaten jeweils ermittelten Ähnlichkeitsgrößen wenigstens eine Anlagen-Inspektionsgröße ermittelt.

Dabei ist die ermittelte Anlagen-Inspektionsgröße charakteristisch für eine Abweichung wenigstens eines Betriebszustands der Behältnisbehandlungsanlage und/oder der/einer Behandlungseinrichtung von einem Normalzustand und/oder einem gewünschten Zustand.

Zusätzlich oder alternativ ist die ermittelte Anlagen-Inspektionsgröße charakteristisch für eine Alterung wenigstens eines Elements der Behältnisbehandlungsanlage und/oder (der/)einer Behandlungseinrichtung und/oder einen in der Behältnisbehandlungsanlage und/oder (der/)einer Behandlungseinrichtung vorliegenden Fehlerzustand.

Dies bietet den Vorteil, dass mögliche Fehler oder eine Alterung (wie Verschleiß) bzw. Fehlerzustände in der Behältnisbehandlungsanlage bereits erkannt werden können, bevor sie sich tatsächlich als (von der Echtzeit-Auswertungseinrichtung bzw. mittels des Behältnisinspektions-Modells erkannte) Defekte in den Behältnisteilen auswirken.

Somit kann in Abhängigkeit der ermittelten Anlagen-Inspektionsgröße eine geeignete Gegenmaßnahme und/oder Steuerungen/Regelungen wenigstens eines Prozesses in der Behältnisbehandlungsanlage vorgenommen werden, welche der als nachteilig angesehenen beobachteten Entwicklung in der Behältnisbehandlungsanlage entgegenwirken kann. Bevorzugt kann ein sogenanntes "predictive maintenance" in Abhängigkeit der Anlagen-Inspektionsgröße und/oder in Abhängigkeit der ermittelten Ähnlichkeitsgrößen durchgeführt werden.

Denkbar ist auch, dass in Abhängigkeit der ermittelten Anlagen-Inspektionsgröße und/oder in Abhängigkeit der ermittelten Ähnlichkeitsgrößen ein Hinweis an einen Bediener ausgegeben wird, dass ein Wartungsintervall verkürzt und/oder eine Wartung durchgeführt werden soll.

Die zur Vielzahl von erfassten Sensordaten jeweils ermittelten Ähnlichkeitsgrößen werden dabei bevorzugt hinsichtlich ihrer in dem Behältnisteil-Strom auftretenden relativen Häufigkeit (bei der Ermittlung einer Anlagen-Inspektionsgröße) ausgewertet.

Denkbar ist, dass es sich bei der Anlagen-Inspektionsgröße um eine statistische Größe handelt, bei welcher beispielsweise die Ähnlichkeitsgrößen zu Sensordaten in Bezug auf mehr als 1000 Behältnisteile, bevorzugt mehr als 10.000, bevorzugt mindestens 50.000 und besonders bevorzugt mindestens 100.000 Behältnisteile berücksichtigt werden/sind. Hierdurch wird vorteilhaft erreicht, dass eine Steuerung/Regelung der Behältnisbehandlungsanlage nur auf Grundlage einer statistisch signifikanten Anlagen-Inspektionsgröße vorgenommen wird.

Bevorzugt wird für das Trainieren der Netze eine Anzahl von markierten und/oder klassifizierten Sensordaten ( beispielsweise Bilder) pro Anwendung der Größenordnung von 1.000 bis 100.000 (beispielsweise 10.000 markierte und/oder klassifizierte Bilder pro Anwendung) verwendet.

Bevorzugt werden die in Bezug auf vorgegebene Referenzdaten ermittelte Ähnlichkeitsgrößen, wie beispielsweise die jeweiligen Abstände der Merkmalsvektoren der jeweils erfassten Sensordaten zu den Merkmalsvektoren der Referenzdaten, mittels (automatischen, bzw. Klbasierten) Mustererkennungsverfahren auf auftretende Muster hin untersucht. Auf diese Weise können etwa (gehäuft) auftretende (insbesondere unbekannte) Besonderheiten in den erfassten Sensordaten und damit in Bezug auf die Behältnisteile erkannt werden. Bevorzugt wird auf derart erkannte Besonderheiten durch etwa Ablegen und Auswerten der Behandlungsdaten und/oder Zustandsdaten der die jeweiligen Behältnisteile behandelnden Behandlungseinrichtungen, welche zum jeweiligen Behandlungszeitpunkt vorlagen, reagiert. Bevorzugt wird das Auswertungsergebnis bei der Steuerung und/oder Regelung der Behältnisbehandlungsanlage berücksichtigt.

Denkbar ist auch, dass die zu einer Vielzahl von erfassten Sensordaten ermittelten Merkmalsvektoren mittels Clusteranalyse (etwa k-Means-Algorithmus) eine Vielzahl von Cluster gebildet werden, welchen die ermittelten Merkmalsvektoren zugeordnet werden/sind und/oder zuordenbar sind. Dabei ist denkbar, dass die hieraus erhaltenen Cluster hinsichtlich bekannter Defekte und/oder Merkmalen von den zu inspizierenden Behältnisteilen ausgewertet werden. So kann etwa ein erhaltener Cluster fehlerfreie Behältnisteile abbilden. Andere erhaltene Cluster können gruppierte Sensordaten repräsentieren, welche jeweils ein Behältnisteil mit einem (gleichen) bekannten Defekt abbilden.

Weiter können hierdurch Cluster erhalten werden, die die erfassten Sensordaten von Behältnisteilen gruppieren, welche keinen bekannten Defekt umfassen. Für einen solchen Cluster charakteristische Daten, beispielsweise ein Centroid bzw. ein Clusterzentrum (und/oder eine Varianz) können an einen Bediener ausgegeben und/oder auf noch unbekannte Defekte und/oder Besonderheiten/Eigenschaften der inspizierten Behältnisteile hin ausgewertet werden.

Denkbar ist auch, dass durch Clusteranalyse (insbesondere in dem Merkmalsraum) erhaltene Centroiden bzw. Clusterzentren als Referenzdaten verwendet werden.

Bei einem weiter bevorzugten Verfahren wird in Abhängigkeit des Inspektionsergebnisses der Anlagen-Inspektionsaufgabe wenigstens eine Behältnisinspektionsaufgabe geändert und/oder angepasst und/oder ergänzt. Auf diese Werte können etwa Schwellenwerte zur Erkennung von Defekten der Behältnisteile fein eingestellt und/oder um neu erkannte Fehlertypen ergänzt werden.

Bei einem weiter bevorzugten Verfahren ist die Ähnlichkeitsgröße charakteristisch für eine Ähnlichkeit der erfassten, insbesondere ortsaufgelösten, Sensordaten zu einer vorgegebenen und/oder vorgebbaren Vielzahl von, insbesondere ortsaufgelösten, Referenz-Sensordaten. Es kann also auch eine Ähnlichkeitsgröße betrachtet werden, die eine Ähnlichkeit zu mehreren Referenz-Sensordaten angibt oder hierfür charakteristisch ist.

Bei einem weiter bevorzugten Verfahren werden die Referenzdaten, bevorzugt die insbesondere ortsaufgelösten Referenz-Sensordaten und/oder die Vielzahl von insbesondere ortsaufgelösten Referenz-Sensordaten, von einem Bediener der Behältnisbehandlungsanlage, bevorzugt mittels einer Mensch-Maschinen-Schnittstelle der Behältnisbehandlungsanlage, vorgegeben. Dies bietet den Vorteil, dass ein Bediener, beispielsweise mittels einer (etwa als Touch-Display ausgebildeten) Eingabeeinrichtung (der Behältnisbehandlungsanlage) dem Bediener (etwa von der Inspektionsvorrichtung und/oder der Behältnisbehandlungseinrichtung), insbesondere durch Anzeigen mittels einer Anzeigeeinrichtung) vorgeschlagene erfasste Sensordaten als Referenz-Sensordaten auswählen kann.

Denkbar ist aber auch, dass der Bediener über die Mensch-Maschine-Schnittstelle der Behältnisinspektionsvorrichtung und/oder der Behältnisbehandlungsanlage die Referenzdaten übermitteln kann, in Bezug auf welche die Ähnlichkeitsgröße zu ermitteln ist. Auf diese Weise können auf benutzerfreundliche Weise Referenzdaten festgelegt werden.

Bei einem weiter bevorzugten Verfahren handelt es sich bei der Behältnisinspektionsaufgabe um eine Klassifikationsaufgabe, welche ausgewählt ist aus einer Gruppe von Klassifikationsaufgaben, welche eine (bevorzugt binäre) Klassifikation in defekte und/oder defektfreie Behältnisteile und bevorzugt Behältnisse (gute / schlechte Behältnisse), eine Erkennung und/oder Klassifikation von Defektarten des Behältnisteils und bevorzugt des Behältnisses, eine Erkennung und/oder Klassifikation von verschiedenen Sorten des Behältnisteils und bevorzugt des Behältnisses (etwa, beispielsweise zehn, verschiedene Flaschentypen), eine Erkennung und/oder Klassifikation einer Kontur und/oder Farbe des Behältnisteils und bevorzugt des Behältnisses, eine Erkennung und/oder Klassifikation einer fehlerfreien und/oder fehlerbehafteten Durchführung wenigstens eines, an dem inspizierten Behältnisteil, insbesondere durch die (erste) Behandlungseinrichtung, vorgenommenen Behandlungsschritts, eine Identifizierung in dem Behältnisteil-Strom vergleichsweise selten auftretender Behältnisteil-Sorten und/oder Defektarten (selten meint insbesondere weniger als 1/1000), eine Etikettenkontrolle, eine Füllhöhenkontrolle, eine Überprüfung eines Schäumungsverhaltens einer Flüssigkeit in einem Behältnis, eine Erkennung eines Haarrisses und/oder Mündungsbruchs eines Behältnisses und/oder Bruchs in einem Bodenbereich des Behältnisses, eine Erkennung von in oder an dem Behältnisteil und/oder Behältnis angeordneten oder befindlichen Fremdpartikeln, und dergleichen sowie Kombinationen hiervon umfasst.

Bevorzugt basiert das Behältnisinspektions-Modell maschinellen Lernens auf einem (künstlichen) neuronalen Netzwerk. Bevorzugt ist das neuronale Netzwerk als tiefes neuronales Netzwerk (Deep Neural Network, DNN), bei dem die parametrierbare Verarbeitungskette eine Mehrzahl von Verarbeitungsschichten aufweist, und/oder ein sogenanntes Convolutional Neural Network (CNN) (dt. Faltungsnetzwerk) und/oder ein rekurrentes Neuronales Netzwerk (RNN, engl. Recurrent Neural Network) und/oder andere DNN layers Klassen ausgebildet.

Bevorzugt handelt es sich bei dem das Behältnisinspektions-Modell maschinellen Lernens, welches von der Echtzeit-Auswertungseinrichtung verwendet wird, um ein bereits angelerntes bzw. (fertig) trainiertes Behältnisinspektions-Modell. Mit anderen Worten liegt das mit dem zu erzeugenden Trainingsdatensatz zu trainierende bzw. genauer nachzutrainierende Behältnisinspektions-Modell in einem Zustand nach Abschluss eines Trainingsvorgangs vor. So ist denkbar, dass das Behältnisinspektions-Modell bereits mit einem allgemeinen, bevorzugt von einer spezifischen bzw. konkreten Behältnisbehandlungsanlage unabhängigen Trainingsdatensatz trainiert wurde/ist.

Bevorzugt werden dem Behältnisinspektions-Modell bzw. dem (künstlichen) neuronalen Netzwerk die (zu verarbeitenden) Daten, insbesondere die ortsaufgelösten Sensordaten (oder hiervon abgeleitete Daten), als Eingangsgrößen zugeführt. Bevorzugt bildet das Behältnisinspektions-Modell bzw. das künstliche neuronale Netzwerk die Eingangsgrößen in Abhängigkeit einer parametrierbaren Verarbeitungskette auf Ausgangsgrößen ab, wobei als Ausgangsgröße bevorzugt eine Behältnisinspektions-Kategorie oder bevorzugt als Ausgangsgrößen eine Vielzahl von Behältnisinspektions-Kategorien gewählt sind.

Bevorzugt es sich bei der Vielzahl von (einander verschiedenartigen) Behältnisinspektions-Kategorien um verschiedene Defektarten des Behältnisteils handeln. Beispielsweise können sich einander verschiedene Behältnisinspektions-Kategorien auf verschiedene Bereiche des Behältnisteils beziehen, welche beispielsweise ausgewählt sind aus einer Gruppe, welche einen Bodenbereich, eine Außenwandungsbereich, einen Seitenwandungsbereich, eine Riffelung im Bodenbereich, einen Tragringbereich, ein Mündungsbereich und dergleichen sowie Kombinationen hiervon umfasst.

Die verschiedenen Behältnisinspektions-Kategorien können sich auch je nach Behältnisinspektionsaufgabe, wie einer Behältnisteil-Boden-, Dichtflächen-, Seitenwand-, Gewinde-, Verschluss-, Füllhöhen-, Etiketten-, Originalitäts-, Schwebstoff, Restflüssigkeitskontrolle ergeben.

Zusätzlich oder alternativ kann sich eine Behältnisinspektions-Kategorie auch auf (genau oder wenigstens) eine von (einander) verschiedenen Defekttypen (etwa Riss, Anriss, Bruch etc.) und/oder Störungsarten beziehen, welche ausgewählt sein können aus einer Gruppe, welche Störstellen, Brüche, Risse, verschiedene Rissarten, (Glas-)Scherben, Abplatzungen, Verschmutzungsstellen, Verschmutzungsarten, Materialverteilungen in Bezug auf einen Defekt und dergleichen sowie Kombinationen hiervon umfasst.

Bevorzugt wird das Behältnisinspektions-Modell maschinellen Lernens bzw. das künstliche neuronale Netzwerk unter Verwendung vorgegebener Trainingsdaten trainiert, wobei durch das Training die parametrierbare Verarbeitungskette parametriert wird.

Bei einem bevorzugten Verfahren werden in dem Trainingsprozess des Behältnisinspektions-Modells Trainingsdaten verwendet, welche eine von der wenigstens einen Sensoreinrichtung erfasste Vielzahl von ortsaufgelösten Sensordaten (von Behältnissen) umfassen. Dies bietet den Vorteil, dass bereits der Trainingsprozess spezifisch auf die einzustellende Behältnisinspektionsvorrichtung abgestimmt wird und beispielsweise damit spezifische Gegebenheiten der spezifischen Behältnisinspektionsvorrichtung, wie optische Eigenschaften der Sensoreinrichtung oder auch spezifische Lichtverhältnisse in der Behältnisinspektionsvorrichtung, unmittelbar berücksichtigt werden können.

Bevorzugt sind/werden die zur Verwendung als Trainingsdaten vorgesehenen (von der wenigstens einen Sensoreinrichtung erfassten) ortsaufgelösten Sensordaten mit (Behältnis- )Sorten- und/oder Klassifikationsmerkmalen versehen. Bevorzugt werden die ortsaufgelösten Sensordaten zusammen mit den jeweils ihnen zugeordneten (Behältnis-)Sorten- und/oder Klassifikationsmerkmalen als Trainingsdatensatz (insbesondere auf einer und/oder der nicht-flüchtigen Speichereinrichtung) abgelegt. Bevorzugt wird auf diese Weise eine Vielzahl von Trainings-Datensätzen erzeugt. Bei den Klassifikationsmerkmalen kann es sich dabei um die (obig beschriebenen) Behältnisinspektions-Kategorien handeln. So können etwa die einem Behältnis zugeordneten ortsaufgelösten Sensordaten mit den darin vorkommenden Defektarten und dergleichen klassifiziert sein.

Bei der nicht-flüchtigen Speichereinrichtung kann es sich um eine Speichereinrichtung handeln, welche (fester) Bestandteil der wenigstens einen Sensoreinrichtung und/oder der Behältnisinspektionsvorrichtung ist. Denkbar ist, dass es sich bei dieser Speichereinrichtung um einen Ringspeicher handelt, bei welchem bei Erreichen der Speicherkapazität die ältesten Sensordaten überschrieben werden (und somit die abgelegten Sensordaten nur für einen begrenzten Zeitraum zur Verfügung stehen).

Bei der nicht-flüchtigen Speichereinrichtung kann es sich ebenfalls um eine Speichereinrichtung der Behältnisbehandlungsanlage handeln, welche etwa als Festspeicher ausgebildet ist.

"Nicht-flüchtig" kann auch heißen, dass die selektierten Bilder bzw. Sensordaten und/oder die auf der nicht-flüchtigen Speichereinrichtung gespeicherten Sensordaten nur über einen gewissen Zeitraum gehalten werden, also beispielsweise nach einer einstellbaren Zeit oder Parametrieraktion (Monteur) gelöscht werden (können). "Nicht-flüchtig" kann auch heißen, dass die Bilder bzw. Sensordaten nicht über das Ausschalten der Maschine hinaus gehalten werden bzw. gespeichert bleiben müssen.

"Nicht-flüchtig" soll auch bedeuten, dass die zu "haltenden" Bilddaten und/oder die abzulegenden Sensordaten im einfachsten Fall für eine Parametrierung zur Verfügung stehen müssen.

Denkbar ist auch, dass zusätzlich oder alternativ unter "nicht-flüchtiger Speichereinrichtung" verstanden wird, dass die zu "haltenden" Bilddaten und/oder die abzulegenden Sensordaten auch erhalten bleiben, wenn die Speichereinrichtung nicht mit Strom versorgt ist.

Das Trainieren der Netze erfordert typischerweise 10.000 oder mehr markierte und/oder klassifizierte Bilder/Sensordaten pro Anwendung. Diese Markierung und/oder Klassifizierung kann lokal oder zentral von Bildverarbeitungs-Experten erfolgen.

Bevorzugt handelt es sich bei dem Arbeitsbetrieb um einen laufenden (Produktions-)Betrieb der Behältnisinspektionsvorrichtung und/oder einen laufenden (Produktions-)Betrieb einer Behältnisbehandlungsanlage wie etwa einer Behältnisabfüllanlage, welche die Behältnisinspektionsvorrichtung aufweist. Insbesondere kann es sich bei dem Arbeitsbetrieb um einen Produktionsbetrieb handeln. Insbesondere handelt es sich bei dem Arbeitsbetrieb nicht um einen Testbetrieb und/oder Wartungsbetrieb und/oder Einstellbetrieb mit etwa gegenüber einer Transportgeschwindigkeit in einem Arbeitsbetrieb verringerten Transportgeschwindigkeit der Behältnisse bzw. Behältnisteile (bei ihrem Durchlauf durch die Behältnisinspektionsvorrichtung).

Bevorzugt ist die Sensoreinrichtung ausgewählt aus einer Gruppe, die eine Bildaufnahmeeinrichtung, etwa eine Kamera (bevorzugt Schwarz-weiß und/oder farbig), einen CMOS-Sensor (CMOS Abk. für Complementary metal-oxide-semiconductor, engl. für "komplementärer / sich ergänzender Metall-Oxid-Halbleiter), einen CCD-Sensor, einen 3D-Sensor, eine Bildaufnahmeeinrichtung auf Röntgen-Basis, ein optisches Element, eine Wärmebildkamera, eine Stereo-Kamera, eine LIDAR-Kamera, ein Geruchssensor und/oder ein (Massen-)Spektrometer, und dergleichen sowie Kombinationen umfasst.

In einer bevorzugten Ausführungsform transportiert die Transporteinrichtung die Behältnisse von einer ersten Behandlungseinrichtung zu einer weiteren (oder zweiten) Behandlungseinrichtung (und/oder ist insbesondere hierfür geeignet und bestimmt).

Bevorzugt ist die erste und/oder die weitere Behandlungseinrichtung ausgewählt aus einer Gruppe, welche eine Spritzgießvorrichtung zum Herstellen eines Spritzgießteils (etwa Vorformlings), eine Reinigungsvorrichtung zum Reinigen der Behältnisse und/oder Behältnisteile, eine Zerkleinerungseinrichtung zum Zerkleinern der Behältnisse, eine Füllvorrichtung zum Füllen der Behältnisse, eine Umformungsvorrichtung zum Umformen eines Kunststoffvorformlings in ein Kunststoffbehältnis, insbesondere eine Blasmaschine, eine Verschließeinrichtung zum Verschließen der Behältnisse, eine Etikettiervorrichtung, eine Markierungseinrichtung, eine Sortiereinrichtung, eine Verpackungseinrichtung (zum Einpacken und/oder Schrumpfverpacken), eine Einrichtung zum Umreifen eines Behältnisteils und/oder Behältnisses, eine Bestimmungseinrichtung zur Bestimmung der Zusammensetzung von Stoffen oder Stoffgemischen, etwa der Atmosphäre und/oder Luft in oder an einem Behältnisteil und bevorzugt Behältnis, beispielsweise ein Massenspektrometer und/oder eine Geruchssensoreinrichtung, und dergleichen sowie Kombinationen hiervon umfasst.

Bevorzugt handelt es sich bei dem Behältnisteil-Strom um einen (insbesondere kontinuierlichen) Strom von (auf dem Transportpfad) sukzessiv bzw. aufeinanderfolgender Behältnisteilen. Beispielsweise kann es sich bei dem Behältnisteil-Strom um einen Behältnisstrom handeln, nämlich einem Strom von (auf dem Transportpfad) sukzessiv bzw. aufeinanderfolgenden Behältnissen. Dabei kann der Behältnisteil-Strom bereichsweise und bevorzugt innerhalb der gesamten Behältnisinspektionsvorrichtung (als Massenstrom) einbahnig oder aber auch mehrbahnig (mittels der Transporteinrichtung) geführt bzw. transportiert werden. Bevorzugt ist wenigstens je eine Sensoreinrichtung einer Bahn des Behältnisteil-Stroms zugeordnet und erfasst jedes auf dieser Bahn befindliche Behältnisteil des Behältnisteil-Stroms.

Bei der Transporteinrichtung kann es sich dabei auch um einen Massentransporteur zum, bevorzugten mehrbahnigen und/oder ungeordneten, Transport einer Vielzahl von Behältnisteilen und bevorzugt von Behältnissen handeln. Bei der Transporteinrichtung kann es sich dabei auch um einen Pufferbereich zum, bevorzugt mehrbahnigen und/oder ungeordneten, Puffern einer Vielzahl von Behältnisteilen und bevorzugt von Behältnissen handeln.

Die Behältnisteile und bevorzugt die Behältnisse können, bevorzugt wenigstens abschnittsweise und bevorzugt entlang in dem gesamten Transportbereich, stehend bzw. aufrecht (von der Transporteinrichtung) transportiert bzw. geführt werden.

Bevorzugt ist die Transporteinrichtung zum wenigstens abschnittsweisen Führen bzw. Transportieren der Vielzahl von Behältnisteilen und bevorzugt von Behältnissen, bevorzugt entlang des gesamten Transportbereichs, von unter Staudruck stehenden Behältnisteilen und bevorzugt Behältnissen geeignet und bestimmt.

Bevorzugt ist die Transporteinrichtung zum Transportieren und/oder Führen (zumindest innerhalb dem Transportbereich) von mindestens 1 Behältnisteil (und bevorzugt mindestens ein Behältnis) pro Stunde, bevorzugt mindestens 5000 (insbesondere zu inspizierende) Behältnisteile (und bevorzugt Behältnisse) pro Stunde, bevorzugt mindestens 20.000 (insbesondere zu inspizierende) Behältnisteile (und bevorzugt Behältnisse) pro Stunde, bevorzugt mindestens 100.000 (insbesondere zu inspizierende) Behältnisteile (und bevorzugt Behältnisse), bevorzugt mindestens 140.000 (insbesondere zu inspizierende) Behältnisteile (und bevorzugt Behältnisse) und besonders bevorzugt mindestens 180.000 (insbesondere zu inspizierende) Behältnisteile (und bevorzugt Behältnisse) geeignet und bestimmt und nimmt dies innerhalb des Arbeitsbetriebs der Behandlungseinrichtung und/oder Behältnisbehandlungsanlage vor. Bevorzugt ist die Transporteinrichtung zum Transportieren und/oder Führen (zumindest innerhalb dem Transportbereich) von höchstens 180.000 (besonders bevorzugt höchstens 200.000), insbesondere zu inspizierenden, Behältnisteilen (und bevorzugt Behältnissen) pro Stunde geeignet und bestimmt und nimmt dies innerhalb des Arbeitsbetriebs der Behandlungseinrichtung und/oder der Behältnisbehandlungsanlage und/oder der Behältnisinspektionsvorrichtung vor.

Bevorzugt ist die Transporteinrichtung in einem einbahnigen Transportbereich zum Transportieren und/oder Führen (zumindest innerhalb dem einbahnigen Transportbereich) von mindestens 100.000 Behältnisteilen (und bevorzugt Behältnissen) pro Stunde und/oder bis zu 1850.000 (besonders bevorzugt höchstens 200.000) Behältnisteilen (und bevorzugt Behältnissen) pro Stunde geeignet und bestimmt und nimmt dies innerhalb des Arbeitsbetriebs der Behandlungseinrichtung und/oder der Behältnisbehandlungsanlage und/oder der Behältnisinspektionsvorrichtung vor.

Bei den Behältnissen kann es sich um Vorformlinge handeln, aus welchen durch einen Umformvorgang fertig ausgeformte Behältnisse hergestellt werden und/oder welche bisher nur durch einen Urformschritt hergestellt sind. Bei den Behältnissen kann es sich auch um bereits fertig ausgeformte Behältnisse handeln, welche beispielsweise durch einen Umformvorgang eines Vorformlings (etwa eines Spritzgussteils bzw. eines Spritzlings) ihre fertige Ausformung erreicht haben.

Bei den Behältnissen kann es sich um leere, noch zu befüllende und/oder zu recycelnde und/oder wieder zu befüllende Behältnisse handeln. Bei den Behältnissen kann es sich auch um befüllte Behältnisse handeln. Zusätzlich oder alternativ kann es sich bei den Behältnissen um mit (insbesondere genau) einem Behältnisverschluss verschlossene und/oder verschließbare Behältnisse handeln.

Bei den Behältnissen kann es sich um Einwegbehältnisse oder um Mehrwegbehältnissen handeln.

Bei den Behältnissen handelt es sich bevorzugt um (bevorzugt dichte), insbesondere verschließbare, Behältnisse zur Aufnahme von Flüssigkeiten und/oder fließfähigen Substanzen, etwa pastöse und/oder cremeartige und/oder gelartige Substanzen, wie etwa aus dem Lebensmittelbereich, aus der Kosmetikindustrie oder aus dem pharmazeutischen Bereich.

Denkbar ist auch, dass es sich bei den Behältnissen um Behältnisse zur Aufnahme von Flüssigkeiten und/oder Körpern handelt, wie etwa für Behältnisse zur Aufnahme von Kontaktlinsen.

Bevorzugt handelt es sich bei der externen Speichereinrichtung um eine Cloud-basierte (bevorzugt nicht-flüchtigen) Speichereinrichtung und/oder einen externen Server (inkl. Speichereinrichtung), wobei auf die Speichereinrichtung insbesondere über das Internet (und/oder über ein, insbesondere wenigstens abschnittsweise drahtgebundenes und/oder drahtloses, öffentliches und/oder privates Netzwerk) zugegriffen wird. Unter einem externen Server ist insbesondere ein in Bezug auf eine Behältnisbehandlungsanlage und/oder Echtzeit-Auswertungseinrichtung externer Server, insbesondere ein Backend-Server, zu verstehen.

Der externe Server ist beispielsweise ein Backend insbesondere eines Behältnisbehandlungsanlagen-Herstellers oder eines Dienstanbieters, welcher dazu eingerichtet ist, ortsaufgelöste Sensordaten (insbesondere einer Vielzahl von Sensoreinrichtungen und/oder einer Vielzahl von Behältnisinspektionsvorrichtungen) zu verwalten und/oder maschinelle Lernverfahren in Bezug auf durchzuführende (Trainings-)Behältnisinspektionsaufgaben durchzuführen und/oder Echtzeit-Auswertungseinrichtungen einzustellen und/oder anzupassen. Die Funktionen des Backend bzw. des externen Servers können dabei auf (externen) Serverfarmen durchgeführt werden. Beim (externen) Server kann es sich um ein verteiltes System handeln.

Die vorliegende Erfindung ist weiterhin gerichtet auf eine Steuervorrichtung für eine Behältnisbehandlungsanlage zur Behandlung einer Vielzahl von Behältnisteilen für Behältnisse und bevorzugt für Kunststoffbehältnisse und/oder Flaschen.

Dabei weist die Behältnisbehandlungsanlage eine Transporteinrichtung zum Transportieren der Vielzahl von Behältnisteilen als Behältnisteil-Strom entlang eines vorgegebenen Transportpfads von wenigstens einer Behandlungseinrichtung der Behältnisbehandlungsanlage zu wenigstens einer weiteren Behandlungseinrichtung der Behältnisbehandlungsanlage auf. Dabei weist die Behältnisbehandlungsanlage zur Durchführung einer Behältnisinspektionsaufgabe wenigstens eine Sensoreinrichtung auf, welche dazu geeignet und bestimmt ist, insbesondere ortsaufgelöste, Sensordaten und bevorzugt Kamerabilder in Bezug auf die Behältnisteile, bevorzugt optisch, zu erfassen.

Dabei weist die Behältnisbehandlungsanlage eine Echtzeit-Auswertungseinrichtung auf, welche zur Auswertung der insbesondere ortsaufgelösten Sensordaten in Echtzeit mittels eines Behältnisinspektions-Modell maschinellen Lernens geeignet und bestimmt ist, welches einen Satz Parameter umfasst, welche auf Werte eingestellt sind, die als Ergebnis eines maschinellen Lernverfahrens gelernt wurden.

Erfindungsgemäß ist die Steuervorrichtung dazu geeignet und bestimmt ist, die erfassten, insbesondere ortsaufgelösten, Sensordaten in Bezug auf vorgegebene und/oder vorgebbare Referenzdaten durch Ermittlung einer Ähnlichkeitsgröße, welche charakteristisch ist für eine Ähnlichkeit der Sensordaten zu den Referenzdaten, auszuwerten.

Dabei wird bevorzugt die Ähnlichkeitsgröße basierend auf einem vorgegebenen, besonders bevorzugt auf dem maschinellen Lernverfahren basierenden Satz von Behältnisteil-Merkmalen ermittelt.

Erfindungsgemäß ist die Steuervorrichtung dazu geeignet und bestimmt, die Ähnlichkeitsgröße oder eine hiervon abgeleitete Größe zur Steuerung und/oder Regelung der Behältnisbehandlungsanlage bereitzustellen und/oder die Behältnisbehandlungsanlage oder eine der Behandlungseinrichtungen der Behältnisbehandlungsanlage in Abhängigkeit der Ähnlichkeitsgröße oder einer hiervon abgeleiteten Größe zu steuern und/oder regeln.

Es wird also auch im Rahmen der erfindungsgemäßen vorgeschlagen, dass die Sensordaten nicht nur mit dem Behältnisinspektions-Modell ausgewertet werden, sondern dass eine zusätzliche Auswertung der Sensordaten vorgenommen wird, welche auf einer Ähnlichkeitsermittlung der erfassten Sensordaten zu vorgegebenen bzw. vorgebbaren Referenzdaten beruht. Auf diese Weise kann ein an sich als "Black Box" wahrnehmbares KI-basiertes Auswertungsmodell ergänzt werden durch eine Ähnlichkeitsanalyse der abgebildeten Behältnisteile.

Bevorzugt ist die Steuervorrichtung dazu eingerichtet, geeignet und/oder bestimmt, das obig beschriebene Verfahren sowie alle bereits obig im Zusammenhang mit dem Verfahren beschriebene Verfahrensschritte einzeln oder in Kombination miteinander, insbesondere in Bezug auf die Ermittlung der Ähnlichkeitsgröße(n) und/oder einer Anlagen-Inspektionsgröße auszuführen. Umgekehrt kann das Verfahren mit allen im Rahmen der Steuervorrichtung beschriebenen Merkmalen einzeln oder in Kombination miteinander ausgestattet sein.

Die vorliegende Erfindung ist weiterhin gerichtet auf eine Behältnisbehandlungsanlage zur Behandlung einer Vielzahl von Behältnisteilen für Behältnisse. Diese umfasst eine Transporteinrichtung zum Transportieren der Vielzahl von Behältnisteilen als Behältnisteil-Strom entlang eines vorgegebenen Transportpfads von und/oder zu wenigstens einer Behandlungseinrichtung der Behältnisbehandlungsanlage, bevorzugt von der wenigstens einen Behandlungseinrichtung zu wenigstens einer weiteren Behandlungseinrichtung der Behältnisbehandlungsanlage.

Dabei weist die Behältnisbehandlungsanlage zur Durchführung einer Behältnisinspektionsaufgabe wenigstens eine Sensoreinrichtung auf, welche dazu geeignet und bestimmt ist, insbesondere ortsaufgelöste, Sensordaten und bevorzugt Kamerabilder in Bezug auf die Behältnisteile, bevorzugt optisch, zu erfassen.

Dabei weist die Behältnisbehandlungsanlage weiterhin eine Echtzeit-Auswertungseinrichtung auf, welche zur Auswertung der insbesondere ortsaufgelösten Sensordaten in Echtzeit mittels eines Behältnisinspektions-Modell maschinellen Lernens geeignet und bestimmt ist. Dabei umfasst das Behältnisinspektions-Modell maschinellen Lernens einen Satz Parameter, welche auf Werte eingestellt sind, die als Ergebnis eines maschinellen Lernverfahrens gelernt wurden.

Erfindungsgemäß weist die Behältnisbehandlungsanlage eine obig beschriebene Steuervorrichtung auf.

Bevorzugt ist die Behältnisbehandlungsanlage dazu eingerichtet, geeignet und/oder bestimmt, die obig beschriebenen Verfahren zum Betreiben einer Behältnisbehandlungsanlage sowie alle bereits obig im Zusammenhang mit dem Verfahren beschriebene Verfahrensschritte einzeln oder in Kombination miteinander auszuführen. Weiter kann die Behältnisbehandlungsanlage und/oder die Behandlungseinrichtung und/oder die wenigstens eine weitere Behandlungseinrichtung und/oder die Transporteinrichtung wenigstens ein obig beschriebenes Merkmal einzeln oder in Kombination mit weiteren Merkmalen aufweisen bzw. ausgestattet sein.

Die vorliegende Erfindung ist weiterhin gerichtet auf ein Computerprogramm oder Computerprogrammprodukt, umfassend Programmmittel, insbesondere einen Programmcode, welcher zumindest einige der und bevorzugt alle Verfahrensschritte der erfindungsgemäßen Verfahren und bevorzugt eine der beschriebenen bevorzugten Ausführungsformen repräsentiert oder kodiert und zum Ausführen durch eine Prozessoreinrichtung ausgebildet ist.

Die vorliegende Erfindung ist weiterhin gerichtet auf einen Datenspeicher, auf welchem zumindest eine Ausführungsform des erfindungsgemäßen Computerprogramms oder eine bevorzugte Ausführungsform des Computerprogramms gespeichert ist.

Die vorliegende Erfindung wurde in Bezug auf ein Behältnis bzw. Behältnisteile für Behältnisse beschrieben. Dabei ist die vorliegende Erfindung auch übertragbar auf Spritzgussteile (Spritzlinge) oder allgemein auf in einer Behandlungsanlage zu behandelnde Artikel (etwa herzustellende und/oder zu verpackende Kontaktlinsen), deren Behandlungsfortschritt und/oder deren Eigenschaften und/oder (Defekt-/Qualitäts-)Zustände durch wenigstens eine Sensoreinrichtungen (zur Erfassung von insbesondere ortsaufgelösten Sensordaten in Bezug auf jeden einzelnen zu inspizierenden Artikel) kontrolliert wird. Die Anmelderin behält sich vor, hierauf gerichtete Gegenstände ebenfalls zu beanspruchen.

Die vorliegende Erfindung ist weiterhin gerichtet auf ein Verfahren zum Betreiben einer Artikelbehandlungsanlage zur Behandlung einer Vielzahl von Spritzgussteilen und/oder Artikeln, wobei eine Transporteinrichtung die Vielzahl von Spritzgussteilen und/oder Artikeln als Teile-Strom entlang eines vorgegebenen Transportpfads von wenigstens einer Behandlungseinrichtung der Artikelbehandlungsanlage zu wenigstens einer weiteren Behandlungseinrichtung der Artikelbehandlungsanlage transportiert, wobei zur Durchführung einer Artikelinspektionsaufgabe wenigstens eine Sensoreinrichtung, insbesondere ortsaufgelöste, Sensordaten und bevorzugt Kamerabilder in Bezug auf die Spritzgussteile und/oder Artikel, bevorzugt optisch, erfasst und eine Echtzeit-Auswertungseinrichtung die insbesondere ortsaufgelösten Sensordaten in Echtzeit mittels eines Artikelinspektions-Modell maschinellen Lernens, welches einen Satz Parameter umfasst, welche auf Werte eingestellt sind, die als Ergebnis eines maschinellen Lernverfahrens gelernt wurden, auswertet.

Erfindungsgemäß ist ein auf einem und bevorzugt dem maschinellen Lernverfahren basierender Satz von Artikel-Merkmalen vorgegeben und die erfassten, insbesondere ortsaufgelösten, Sensordaten werden in Bezug auf eine, von der Artikelinspektionsaufgabe verschiedene Anlagen-Inspektionsaufgabe basierend auf dem vorgegebenen Satz von Artikel-Merkmalen ausgewertet, wobei in Abhängigkeit des Inspektionsergebnisses der durchgeführten Anlagen-Inspektionsaufgabe wenigstens eine Anlageninspektionsgröße ermittelt, welche zur Steuerung und/oder Regelung der Artikelbehandlungsanlage bereitgestellt wird.

Die vorliegende Erfindung ist weiterhin gerichtet auf eine Steuervorrichtung für eine Artikelbehandlungsanlage zur Behandlung einer Vielzahl von Spritzgussteilen und/oder Artikeln, wobei die Artikelbehandlungsanlage eine Transporteinrichtung zum Transportieren der Vielzahl von Spritzgussteilen und/oder Artikeln als Teile-Strom entlang eines vorgegebenen Transportpfads von wenigstens einer Behandlungseinrichtung der Artikelbehandlungsanlage zu wenigstens einer weiteren Behandlungseinrichtung der Artikelbehandlungsanlage aufweist, wobei die Artikelbehandlungsanlage zur Durchführung einer Artikelinspektionsaufgabe wenigstens eine Sensoreinrichtung aufweist, welche dazu geeignet und bestimmt ist, insbesondere ortsaufgelöste, Sensordaten und bevorzugt Kamerabilder in Bezug auf die Spritzgussteile und/oder Artikel, bevorzugt optisch, zu erfassen, und wobei die Artikelbehandlungsanlage und eine Echtzeit-Auswertungseinrichtung aufweist, welche zur Auswertung der insbesondere ortsaufgelösten Sensordaten in Echtzeit mittels eines Artikelinspektions-Modell maschinellen Lernens geeignet und bestimmt ist, welches einen Satz Parameter umfasst, welche auf Werte eingestellt sind, die als Ergebnis eines maschinellen Lernverfahrens gelernt wurden.

Erfindungsgemäß ist die Steuervorrichtung dazu geeignet und bestimmt, die erfassten, insbesondere ortsaufgelösten, Sensordaten in Bezug auf vorgegebene und/oder vorgebbare Referenzdaten durch Ermittlung einer Ähnlichkeitsgröße, welche charakteristisch ist für eine Ähnlichkeit der Sensordaten zu den Referenzdaten, auszuwerten, wobei bevorzugt die Ähnlichkeitsgröße basierend auf einem vorgegebenen, besonders bevorzugt auf dem maschinellen Lernverfahren basierenden Satz von Artikel-Merkmalen ermittelt wird, wobei die Steuervorrichtung dazu geeignet und bestimmt ist, die Ähnlichkeitsgröße oder eine hiervon abgeleitete Größe zur Steuerung und/oder Regelung der Artikelbehandlungsanlage bereitzustellen.

Die weiteren obig im Rahmen der Behältnisteile beschriebenen Merkmale sind entsprechend analog auf die Spritzgussteile und/oder Artikel anwendbar.

Weitere Vorteile und Ausführungsformen ergeben sich aus der beigefügten Zeichnung:
Darin zeigen:
- Fig. 1: eine schematische Darstellung einer erfindungsgemäßen Behältnisbehandlungsanlage gemäß einer bevorzugten Ausführungsform; und
- Fig. 2: Kamerabilder zur Veranschaulichung ermittelter Ähnlichkeitsgrößen.

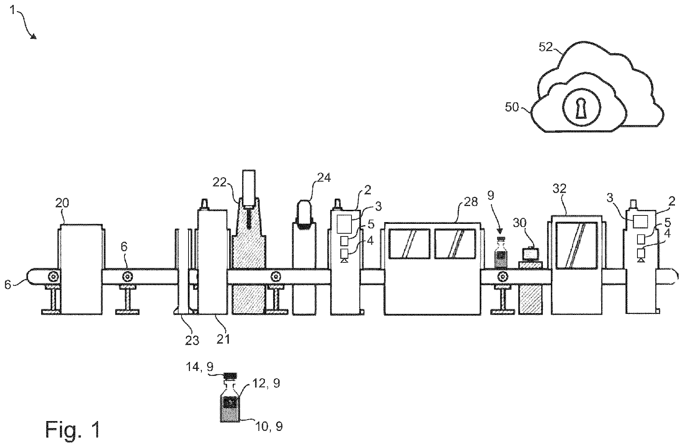

Fig. 1 zeigt eine schematische Darstellung einer erfindungsgemäßen Behältnisbehandlungsanlage 1 zum Behandeln von Behältnisteilen 10, hier als Flaschen ausgebildete Behältnisse 10, gemäß einer ersten Ausführungsform.

Das Bezugszeichen 12 kennzeichnet eine an dem Behältnisteil 10, hier einem Behältnis, angeordnete Ausstattung. In dem in Fig. 1 dargestellten Ausführungsbeispiel ist als Ausstattung ein Identifikationsmittel beispielhaft gezeigt, welches an der Flasche 10 angeordnet ist. Dabei handelt es sich beispielsweise um einen (aufgedruckten) QR-Code. Das Bezugszeichen 14 kennzeichnet einen Behältnisverschluss als eine weitere Ausstattung des Behältnisses 10.

In der in Fig. 1 gezeigten Ausführungsform wird ein Kunststoffvorformling bereitgestellt und von der Transporteinrichtung 6 einer Erwärmungsvorrichtung 20 zugeführt, in dieser erwärmt und im Anschluss in einer Blasformvorrichtung als weitere Behandlungseinrichtung, deren Anordnung innerhalb der Behältnisbehandlungsanlage 1 durch das Bezugszeichen 23 gekennzeichnet ist, zu einer (Kunststoff-)Flasche 10 expandiert. Diese Flasche 10 kann beispielsweise durch die Individualisierungseinrichtung, etwa einer Druckeinrichtung, mit einem Identifikationsmittel 12 versehen sein, wodurch eine Flasche, welche ein Identifikationsmittel 12 aufweist, entsteht.

Das Behältnisteil 9 kann in der Behältnisbehandlungsanlage 1 von wenigstens einer Transporteinrichtung 6 von einer Behandlungseinrichtung zur nächsten, sowie auch innerhalb der Behandlungseinrichtung(en) transportiert werden. Dargestellt sind hier als Behandlungseinrichtungen (in einer Reihenfolge stromabwärts der Transportrichtung der Flasche) eine Inspektionsvorrichtung 21, eine Füllvorrichtung 22 zum Befüllen der Flasche 10 mit einem Produkt, eine Verschließvorrichtung 24, eine Trocknungsvorrichtung 28, eine Etikettiervorrichtung 30 sowie eine Verpackungseinrichtung 32 zum Verpacken der Flasche 10.

Die Bezugszeichen 2 kennzeichnet jeweils eine weitere Behältnisinspektionsvorrichtung (etwa am Ende der Linie und zwischen der Verschließeinrichtung 24 und der Trocknungseinrichtung 28 angeordnet), welche beispielsweise eine Füllhöhe in der Flasche und/oder eine ordnungsgemäße Anordnung des Verschlusses auf der Flasche 10 und/oder einen Sicherungsring und/oder eine ordnungsgemäße Etikettierung und/oder Verpackung der Flasche 10 oder weitere Produktionsdaten überprüft.

Das Bezugszeichen 4 kennzeichnet jeweils eine Sensoreinrichtung, hier einer Kamera, mittels welcher - individuell zu jedem (zu inspizierenden) Behältnisteil 9 - Sensordaten in Bezug auf das jeweilige Behältnisteil 9 erhoben bzw. erfasst bzw. aufgenommen werden.

Das Bezugszeichen 3 kennzeichnet jeweils eine Echtzeit-Auswertungseinrichtung, mittels welcher die von der Sensoreinrichtung 4 der jeweiligen Behältnisinspektionsvorrichtung 2 erfassten Sensordaten zur Durchführung einer (vorgegebenen und/oder festgelegten) Behältnisinspektionsaufgabe ausgewertet werden.

Bei einem bevorzugt vorgeschlagenen Verfahren wird zur Auswertung ein Satz extrahierter Merkmale zur Bewertung der erfassten Sensordaten verwendet. Der verwendete Satz extrahierter Merkmale ist das Ergebnis einer (trainierten) Merkmalsextraktion, welche durch ein neuronales Netz, das mit (umfangreichen) (Trainings-)Daten auf ähnliche (Inspektions-)Aufgaben der Bild-Klassifikation vortrainiert wurde. Im letzten Schritt der Bewertung der extrahierten Merkmale wird dann jedoch ein klassisches Klassifikationsverfahren angewendet.

Das Bezugszeichen 50 kennzeichnet einen internen Server bzw. eine interne Speichereinrichtung und das Bezugszeichen 52 einen externen Server bzw. eine externe, insbesondere Cloud-basierte, Speichereinrichtung. Auf dem externen Server 52 kann beispielsweise die Al-basierte Merkmalsextraktion vorgenommen werden. Auf der externen und/oder der internen Speichereinrichtung 50/52 kann bevorzugt der erhaltene Satz extrahierter Behältnisteil-Merkmale abgelegt werden.

Das Bezugszeichen 5 kennzeichnet eine Speichereinrichtung, welche hier (fester) Bestandteil der Behältnisinspektionsvorrichtung 2 ist. Auf dieser Speichereinrichtung 2 können von der Sensoreinrichtung 4 erfasste Sensordaten abgelegt werden.

Bevorzugt kann als Referenz kann beispielsweise ein Bild (als Referenzdaten) eines bestimmten Fehlers bzw. Merkmals des Behältnisteils verwendet werden. Mit Hilfe einer bevorzugt Al-basierten Ähnlichkeitsmetrik kann die Ähnlichkeit der von der Kamera erfassten Bilder zum Referenzbild bestimmt werden. Damit können auch Kamerabilder gefunden werden, die Behältnisse mit dem gesuchten bestimmten Fehler bzw. Merkmal mit nur einer geringen Ausprägung zeigen, deren zugehörigen Behältnisse aufgrund der nur geringen Ausprägung nicht erkannt und/oder ausgeleitet werden. Werden auf diese beispielsweise (zunehmend gehäuft) mehrere Behältnisse mit bereits geringer Ausprägung dieses Fehlers erkannt, kann dies beispielsweise für eine sich kontinuierlich vergrößernde Abweichung eines Prozessparameters (etwa Temperatur einer Reinigungsflüssigkeit und/oder Temperatur in einer Heizeinrichtung zum Erwärmen der Kunststoffvorformlinge) sprechen.

Bevorzugt kann hierzu die Behältnisinspektionsvorrichtung 2 oder eine Steuervorrichtung eine Ähnlichkeitsgröße ermitteln, welche für eine Ähnlichkeit der erfassten Bilder bzw. Sensordaten zu vorgegebenen und/oder vorgebbaren Referenzdaten (wie einem Referenz-Bild) charakteristisch ist.

In Abhängigkeit von der jeweils ermittelten Ähnlichkeitsgrößen kann eine Anlagen-Inspektionsgröße ermittelt werden, welche charakteristisch für einen (unerwünschten) Ist-Zustand der Behältnisbehandlungsanlage und/oder einer Behältnisbehandlungseinrichtung ist (etwa Temperaturzustand).

In Abhängigkeit dieser Anlagen-Inspektionsgröße kann sodann eine Steuerung/Regelung etwa des Zustands der Behältnisbehandlungsanlage und/oder der Behältnisbehandlungseinrichtung zur Erreichung eines (gewünschten) Soll-Zustands vorgenommen werden.

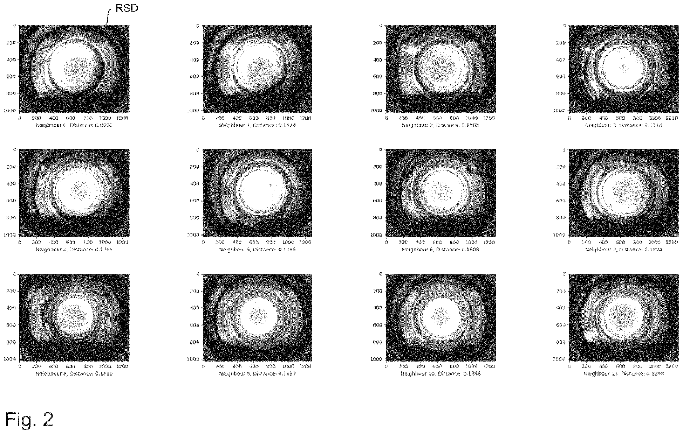

Figur 2 zeigt zwölf Kamerabilder zur Veranschaulichung einer ermittelten Ähnlichkeitsgröße.

Insbesondere wurden die (sowie weitere nicht dargestellte Kamerabilder) zur Bewertung einer Ähnlichkeit verwendet. Fig. 2 zeigt ein Ergebnis der entsprechend ihrer Ähnlichkeit (mit nach absteigender Ähnlichkeit) sortierten Kamerabildern.

Diese Kamerabilder werden im Rahmen einer Bodeninspektion eines Behältnisses von einer Kamera aufgenommen, welche durch die Mündung des Behältnisses hindurch den Boden des Behältnisses inspiziert. Dabei wird der Boden in einem Durchlichtverfahren von einer Beleuchtungseinrichtung beleuchtet.

Das erste Bild, in der Figurenebene der Fig. 2 links oben, welches mit dem Bezugszeichen RSD gekennzeichnet ist, wird dabei als Referenz-Bild verwendet. Dieses weist somit den mittels (etwa einer euklidischen) Abstandsmetrik ermittelten Abstand 0 zu sich selbst auf.

Die weiteren in Fig. 2 gezeigten Kamerabilder sind entsprechend dem jeweiligen in Bezug auf die Abstandsmetrik ermittelten Abstand zu dem Referenz-Bild sortiert (von links nach rechts, dann von oben nach unten), und zeigen damit einen steigenden Abstand, also eine abnehmende Ähnlichkeit.

Das letzte Kamerabild, in der Figurenebene rechts unten angeordnet, hat den im Vergleich zu den anderen in Fig. 2 gezeigten Kamerabildern höchsten Abstand mit einem Abstand von 0,1848 und ist somit der elfte Nachbar des Referenz-Bildes.

Anhand dieser Kamerabilder lässt sich die hohe Leistung des vorgeschlagenen Verfahrens veranschaulichen. Das Referenzbild RSD zeigt einen Behältnisboden mit einem Embossing "BA". Die von dem vorgeschlagenen Verfahren hierzu am ähnlichsten bewerteten Kamerabilde, nämlich der 1. Nachbar ("Neighbour 1") und der 2. Nachbar ("Neighbour 2"), zeigen (mit abnehmender Deutlichkeit) ebenfalls noch ein solches Embossing "BA". Der 3. Nachbar ("Neighbour 3") zeigt mittig einen Tropfen, welcher ebenfalls eine ähnlich runde Form wie eine Innenkontur des "B" aufweist.

Fig. 2 zeigt, dass durch das vorgeschlagene Verfahren, eine Bewertung einer Ähnlichkeit mittels einer Abstandsmetrik in einem Merkmalsraum (wobei der Merkmalsraum durch, in einem Al basierten Trainingsverfahren extrahierten Merkmale aufgespannt ist), in welchem die Bilder jeweils als Merkmalsvektoren dargestellt werden, vorzunehmen, erreicht werden kann, dass alle Behältnisböden mit dem Embossing "BA" unter den nächsten vier Nachbarn sortiert werden können.

Dieses Verfahren gibt die Möglichkeit, Bilder, welche ähnlich einem vorgegebenen Referenzbild sind, aus den erfassten Bildern (eines Behältnisstroms) zu ermitteln. Aus diesem Ermittlungsergebnis kann sodann darauf geschlossen werden, ob die Behältnisbehandlungsanlage einen reibungslosen Ablauf durchführt oder - andererseits - ob in die Steuerung und/oder Regelung eingegriffen werden soll.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

### Bezugszeichenliste

- 1: Behältnisbehandlungsanlage
- 2, 21: Behältnisinspektionsvorrichtung
- 3: Echtzeit-Auswertungseinrichtung
- 4: Sensoreinrichtung
- 6: Transporteinrichtung
- 10: Behältnis
- 9: Behältnisteil
- 12: Ausstattung, Direktdruckelement
- 14: Ausstattung, Behältnisverschluss
- 20: Behandlungseinrichtung, hier Erwärmungseinrichtung
- 23: Behandlungseinrichtung, hier Druckeinrichtung
- 22: Behandlungseinrichtung, hier Fülleinrichtung
- 24: Behandlungseinrichtung, hier Verschließeinrichtung
- 28: Behandlungseinrichtung, hier Trocknungseinrichtung
- 30: Behandlungseinrichtung, hier Etikettiereinrichtung
- 32: Behandlungseinrichtung, hier Verpackungseinrichtung
- 50: interner Server, Speichereinrichtung
- 52: externer Server Speichereinrichtung

## Patentansprüche

1. Verfahren zum Betreiben einer Behältnisbehandlungsanlage (1) zur Behandlung einer Vielzahl von Behältnisteilen (9) für Behältnisse (10) und bevorzugt für Kunststoffbehältnisse und/oder Flaschen, wobei eine Transporteinrichtung (6) die Vielzahl von Behältnisteilen (9) als Behältnisteil-Strom entlang eines vorgegebenen Transportpfads von wenigstens einer Behandlungseinrichtung (20, 23, 22, 24, 28, 30) der Behältnisbehandlungsanlage (1) zu wenigstens einer weiteren Behandlungseinrichtung (23, 22, 24, 28, 30, 32) der Behältnisbehandlungsanlage (1) transportiert, wobei zur Durchführung einer Behältnisinspektionsaufgabe wenigstens eine Sensoreinrichtung (4), insbesondere ortsaufgelöste, Sensordaten und bevorzugt Kamerabilder in Bezug auf die Behältnisteile (9), bevorzugt optisch, erfasst und eine Echtzeit-Auswertungseinrichtung (3) die insbesondere ortsaufgelösten Sensordaten in Echtzeit mittels eines Behältnisinspektions-Modell maschinellen Lernens, welches einen Satz Parameter umfasst, welche auf Werte eingestellt sind, die als Ergebnis eines maschinellen Lernverfahrens gelernt wurden, auswertet,
**dadurch gekennzeichnet, dass**
ein auf einem und bevorzugt dem maschinellen Lernverfahren basierender Satz von Behältnisteil-Merkmalen vorgegeben ist und die erfassten, insbesondere ortsaufgelösten, Sensordaten in Bezug auf eine, von der Behältnisinspektionsaufgabe verschiedene Anlagen-Inspektionsaufgabe basierend auf dem vorgegebenen Satz von Behältnisteil-Merkmalen ausgewertet werden, wobei in Abhängigkeit des Inspektionsergebnisses der durchgeführten Anlagen-Inspektionsaufgabe wenigstens eine Anlageninspektionsgröße ermittelt, welche zur Steuerung und/oder Regelung der Behältnisbehandlungsanlage (1) bereitgestellt wird.

2. Verfahren zum Betreiben einer Behältnisbehandlungsanlage (1) zur Behandlung einer Vielzahl von Behältnisteilen (9) für Behältnisse (10) und bevorzugt für Kunststoffbehältnisse und/oder Flaschen, wobei eine Transporteinrichtung (6) die Vielzahl von Behältnisteilen (9) als Behältnisteil-Strom entlang eines vorgegebenen Transportpfads von wenigstens einer Behandlungseinrichtung (20, 23, 22, 24, 28, 30) der Behältnisbehandlungsanlage (1) zu wenigstens einer weiteren Behandlungseinrichtung (23, 22, 24, 28, 30, 32) der Behältnisbehandlungsanlage (1) transportiert, wobei zur Durchführung einer Behältnisinspektionsaufgabe wenigstens eine Sensoreinrichtung (4), insbesondere ortsaufgelöste, Sensordaten und bevorzugt Kamerabilder in Bezug auf die Behältnisteile (9), bevorzugt optisch, erfasst und eine Echtzeit-Auswertungseinrichtung (3) die insbesondere ortsaufgelösten Sensordaten in Echtzeit mittels eines Behältnisinspektions-Modell maschinellen Lernens, welches einen Satz Parameter umfasst, welche auf Werte eingestellt sind, die als Ergebnis eines maschinellen Lernverfahrens gelernt wurden, auswertet,
**dadurch gekennzeichnet, dass**
die erfassten, insbesondere ortsaufgelösten, Sensordaten in Bezug auf vorgegebene und/oder vorgebbare Referenzdaten durch Ermittlung einer Ähnlichkeitsgröße, welche charakteristisch ist für eine Ähnlichkeit der Sensordaten zu den Referenzdaten, ausgewertet werden, wobei bevorzugt die Ähnlichkeitsgröße basierend auf einem vorgegebenen, besonders bevorzugt auf dem maschinellen Lernverfahren basierenden Satz von Behältnisteil-Merkmalen ermittelt wird, wobei die Ähnlichkeitsgröße oder eine hiervon abgeleitete Größe zur Steuerung und/oder Regelung der Behältnisbehandlungsanlage (1) bereitgestellt wird.

3. Verfahren nach Anspruch 1 oder 2, wobei es sich bei dem Satz von Behältnisteil-Merkmalen um einen, im Rahmen des maschinellen Lernverfahrens erhaltenen Satz von, insbesondere von einem neuronalen Netz, extrahierten Behältnisteil-Merkmalen handelt.

4. Verfahren nach dem vorangegangenen Anspruch, **dadurch gekennzeichnet, dass** es sich bei dem Lernverfahren, im Rahmen dessen der Satz von Behältnisteil-Merkmalen extrahiert wird, um ein überwachtes Lernverfahren, bevorzugt um einen K-nächste-Nachbarn-Algorithmus handelt.

5. Verfahren nach einem der vorangegangenen Ansprüche, **dadurch gekennzeichnet, dass** auf Grundlage der, auf dem Satz von Behältnisteil-Merkmalen basierenden Auswertung der in Bezug auf ein Behältnisteil erfassten Sensordaten eine wenigstens abschnittsweise Nachverfolgung einer Bewegung des Behältnisteils durch die Behältnisbehandlungsanlage (1) vorgenommen wird.

6. Verfahren nach einem der vorangegangenen Ansprüche, **dadurch gekennzeichnet, dass** die Nachverfolgung des Behältnisteils vorgenommen wird, ohne dass ein zur Individualisierung vorgesehener Behandlungsschritt an dem Behältnisteil vorgenommen wurde und/oder ist.

7. Verfahren nach einem der vorangegangenen Ansprüche, **dadurch gekennzeichnet, dass** basierend auf dem Satz von Behältnisteil-Merkmalen die in Bezug auf ein Behältnisteil (9) erfassten Sensordaten dahingehend ausgewertet werden, dass wenigstens eine für das Behältnisteil (9) charakteristische Identifikationsgröße ermittelt wird.

8. Verfahren nach einem der vorangegangenen Ansprüche, **dadurch gekennzeichnet, dass** auf Grundlage der Auswertung der in Bezug auf ein Behältnisteil erfassten Sensordaten mittels dem Satz von Behältnisteil-Merkmalen überprüft wird, ob ein individuelles Behältnisteil (9) wiederholt die Sensoreinrichtung (4) erreicht hat.

9. Verfahren nach dem vorhergehenden Anspruch, **dadurch gekennzeichnet, dass** zur Überprüfung einer wiederholten Erreichung der das individuelle Behältnisteils (9) erfassenden Sensoreinrichtung (4) basierend auf dem Satz von Behältnisteil-Merkmalen eine für das Behältnisteil (9) charakteristische Identifikationsgröße ermittelt und auf Grundlage der ermittelten Identifikationsgröße eine Ähnlichkeit zu nachfolgend von der Sensoreinrichtung (4) erfassten Sensordaten ermittelt wird.

10. Verfahren nach dem vorangegangenen Anspruch, **dadurch gekennzeichnet, dass** bei festgestellter wiederholter Erreichung der Sensoreinrichtung (4) durch ein Behältnisteil (9) ein erneuter Transport des Behältnisteils (9) zu dieser Sensoreinrichtung (4), bevorzugt durch individuelle Änderung wenigstens eines bisherigen Behandlungsschrittes in Bezug auf das Behältnisteil (9) und besonders bevorzugt durch Ausschleusen des Behältnisteils (9) aus dem Behältnisteil-Strom, verhindert wird.

11. Verfahren nach einem der vorangegangenen Ansprüche, **dadurch gekennzeichnet, dass** Referenz-Daten vorgegeben und/oder vorgebbar sind und die Steuerung und/oder Regelung basierend auf ermittelten Ähnlichkeitsgröße, welche charakteristisch ist für eine Ähnlichkeit zwischen den Referenzdaten und in Bezug auf wenigstens ein Behältnisteil (9) und bevorzugt in Bezug auf eine Vielzahl von Behältnisteilen (9) jeweils erfassten Sensordaten.

12. Verfahren nach einem der beiden vorhergehenden Ansprüche, **dadurch gekennzeichnet, dass** wenigstens ein Behandlungsschritt und/oder wenigstens ein Betriebszustand der Behältnisbehandlungsanlage (1) in Abhängigkeit des Inspektionsergebnisses der Anlagen-Inspektionsaufgabe geändert und/oder angepasst wird.

13. Verfahren nach einem der vorangegangenen Ansprüche, **dadurch gekennzeichnet, dass** in Abhängigkeit des Inspektionsergebnisses der Anlagen-Inspektionsaufgabe wenigstens eine Behältnisinspektionsaufgabe geändert und/oder angepasst und/oder ergänzt wird.

14. Steuervorrichtung für eine Behältnisbehandlungsanlage (1) zur Behandlung einer Vielzahl von Behältnisteilen (9) für Behältnisse (10) und bevorzugt für Kunststoffbehältnisse und/oder Flaschen, wobei die Behältnisbehandlungsanlage (1) eine Transporteinrichtung (6) zum Transportieren der Vielzahl von Behältnisteilen (9) als Behältnisteil-Strom entlang eines vorgegebenen Transportpfads von wenigstens einer Behandlungseinrichtung (20, 23, 22, 24, 28, 30) der Behältnisbehandlungsanlage (1) zu wenigstens einer weiteren Behandlungseinrichtung (23, 22, 24, 28, 30, 32) der Behältnisbehandlungsanlage (1) aufweist, wobei die Behältnisbehandlungsanlage (1) zur Durchführung einer Behältnisinspektionsaufgabe wenigstens eine Sensoreinrichtung (4) aufweist, welche dazu geeignet und bestimmt ist, insbesondere ortsaufgelöste, Sensordaten und bevorzugt Kamerabilder in Bezug auf die Behältnisteile (9), bevorzugt optisch, zu erfassen, und wobei die Behältnisbehandlungsanlage und eine Echtzeit-Auswertungseinrichtung (3) aufweist, welche zur Auswertung der insbesondere ortsaufgelösten Sensordaten in Echtzeit mittels eines Behältnisinspektions-Modell maschinellen Lernens geeignet und bestimmt ist, welches einen Satz Parameter umfasst, welche auf Werte eingestellt sind, die als Ergebnis eines maschinellen Lernverfahrens gelernt wurden,
**dadurch gekennzeichnet, dass**
die Steuervorrichtung dazu geeignet und bestimmt ist, die erfassten, insbesondere ortsaufgelösten, Sensordaten in Bezug auf vorgegebene und/oder vorgebbare Referenzdaten durch Ermittlung einer Ähnlichkeitsgröße, welche charakteristisch ist für eine Ähnlichkeit der Sensordaten zu den Referenzdaten, auszuwerten, wobei bevorzugt die Ähnlichkeitsgröße basierend auf einem vorgegebenen, besonders bevorzugt auf dem maschinellen Lernverfahren basierenden Satz von Behältnisteil-Merkmalen ermittelt wird, wobei die Steuervorrichtung dazu geeignet und bestimmt ist, die Ähnlichkeitsgröße oder eine hiervon abgeleitete Größe zur Steuerung und/oder Regelung der Behältnisbehandlungsanlage (1) bereitzustellen.

15. Behältnisbehandlungsanlage (1) zur Behandlung einer Vielzahl von Behältnisteilen (9) für Behältnisse (10) umfassend eine Transporteinrichtung (6) zum Transportieren der Vielzahl von Behältnisteilen (9) als Behältnisteil-Strom entlang eines vorgegebenen Transportpfads von wenigstens einer Behandlungseinrichtung (20, 23, 22, 24, 28, 30) der Behältnisbehandlungsanlage (1) zu wenigstens einer weiteren Behandlungseinrichtung (23, 22, 24, 28, 30, 32) der Behältnisbehandlungsanlage (1), wobei die Behältnisbehandlungsanlage (1) zur Durchführung einer Behältnisinspektionsaufgabe wenigstens eine Sensoreinrichtung (4) aufweist, welche dazu geeignet und bestimmt ist, insbesondere ortsaufgelöste, Sensordaten und bevorzugt Kamerabilder in Bezug auf die Behältnisteile (9), bevorzugt optisch, zu erfassen, und wobei die Behältnisbehandlungsanlage und eine Echtzeit-Auswertungseinrichtung (3) aufweist, welche zur Auswertung der insbesondere ortsaufgelösten Sensordaten in Echtzeit mittels eines Behältnisinspektions-Modell maschinellen Lernens geeignet und bestimmt ist, welches einen Satz Parameter umfasst, welche auf Werte eingestellt sind, die als Ergebnis eines maschinellen Lernverfahrens gelernt wurden, **dadurch gekennzeichnet, dass** die Behältnisbehandlungsanlage (1) eine Steuervorrichtung nach dem vorhergehenden Anspruch aufweist.
